Europäisches Patentamt

European Patent Office

Office européen des brevets

(19)

(11) Publication number: **0 220 520 B1**

# (12) EUROPEAN PATENT SPECIFICATION

(45) Date of publication of patent specification: **18.09.91**    (51) Int. Cl.⁵: **C12N 15/27, C12P 21/02**

(21) Application number: **86113446.8**

(22) Date of filing: **30.09.86**

(54) Human granulocyte colony stimulating factor.

<table>
<tr><td>

(30) Priority: **30.09.85 JP 217150/85**
**17.07.86 JP 166710/86**
**17.07.86 JP 166709/86**

(43) Date of publication of application:
**06.05.87 Bulletin 87/19**

(45) Publication of the grant of the patent:
**18.09.91 Bulletin 91/38**

(84) Designated Contracting States:
**AT BE CH DE FR GB IT LI NL SE**

(56) References cited:
**EP-A- 0 169 566**
**EP-A- 0 183 350**
**WO-A-85/04188**
**WO-A-86/00639**
**WO-A-86/04605**

**Proc.Natl.Acad.Sci. USA, vol. 82, 1985**
**(March), pp. 1526-1530**

</td><td>

(73) Proprietor: **Chugai Seiyaku Kabushiki Kaisha**
**5-1, 5-chome, Ukima Kita-ku**
**Tokyo(JP)**

(72) Inventor: **Yamazaki, Tatsumi**
**5-7-25-224, Kinuta**
**Setagaya-ku Tokyo(JP)**
Inventor: **Nagata, Shigekazu**
**2-24-62-3-305, Tamagawa**
**Ota-ku Tokyo(JP)**
Inventor: **Tsuchiya, Masayuki**
**Azeriahaitsu Seishin 406 2-928, Ikebukuro**
**Toshima-ku Tokyo(JP)**

(74) Representative: **Vossius & Partner**
**Siebertstrasse 4 P.O. Box 86 07 67**
**W-8000 München 86(DE)**

</td></tr>
</table>

## Description

The present invention relates to a human granulocyte colony stimulating factor. More particularly, the present invention relates to a human chromosomal gene coding for a polypeptide having the activity of a colony stimulating factor (hereinafter abbreviated as CSF) which is a specific stimulating factor necessary for the principal purpose of forming colonies of human granulocytic cells. The present invention also relates to a recombinant vector inserted said gene, a transformant containing said vector and a process for producing a glycoprotein having the CSF activity.

When bone marrow cells as target cells and kidney cells or fetal cells were cultured by the double-layer soft agar cultivation method, with the bone marrow cells being in the upper layer and the kidney or fetal cells in the lower layer, part of the cells in the upper layer grew and differentiated to form colonies of neutrophilic granulocytes (hereunder simply referred to as granulocytes) or monocytic macrophages. This observation has led to the assumption of the presence in vivo of factors which promote the formation of colonies [Pluznik and Sach, J. Cell. Comp. Physiol., 66, 319 (1965); and Bradley and Metcalf, Aust. J. Exp. Biol. Med. Sci., 44, 287 (1966)].

These factors which are collectively referred to as CSF are known to be produced by cells, such as T-cells, monocytic macrophages, fibroblasts and endothelial cells, which normally are distributed extensively in vivo. Among subclasses of CSF are included: granulocyte-monocytic macrophage CSF (abbreviated as GM-CSF) which act on the stem cells of granulocytes or monocyte macrophages in such a manner that they stimulate the growth of such stem cells and induce their differentiation to form colonies of granulocytes or monocytic macrophages; monocytic macrophage CSF (abbreviated as M-CSF) which is principally capable of forming colonies of monocytic macrophages; multipotent CSF (abbreviated as multi-CSF) which acts on less differentiated multipotent stem cells; and granulocyte CSF (abbreviated as G-CSF) of the type contemplated by the present invention which is principally capable of forming granulocytic colonies. It has recently been held that the stages of differentiation of target cells differ from one subclass of CSF to another [Asano, Taisha - Metabolism and Disease, 22, 249 (1985); and Yunis et al., "Growth and Maturation Factors", edited by Guroff, John Wiley & Sons, NY, vol. 1, 209 (1983)].

Therefore, purifying the individual CSF subclasses and making a closer study of their chemical and biological properties are very important for the purpose of estimating the hematopoietic mechanisms and analyzing the pathomorphological aspects of various hematological diseases. The biological actions of G-CSF that are drawing increasing attention of researchers are their capabilities of inducing the differentiation of bone marrow leukemic cells and enhancing the functions of mature granulocytes, and much promise has been held in the potential clinical utility of G-CSF in the fields of treating and preventing leukemia.

The attempts heretofore made to isolate and purify G-CSF are based on the method of cell cultivation wherein G-CSF is isolated from the supernatant of a cell culture, but homogeneous G-CSF has yet to be produced in large quantities by this method because G-CSF can only be produced in low concentration and complex purification procedures are required to obtain a trace amount of G-CSF from a large volume of culture solution. Therefore, it has been strongly desired to achieve mass production of G-CSF by recombinant DNA technology.

One object of the present invention is to provide a human chromosomal gene encoding a polypeptide having human G-CSF activity.

Another object of the present invention is to provide a recombinant vector having inserted said gene.

Still another object of the present invention is to provide a transformant which has been produced by transforming a host with said recombinant vector.

A further object of the present invention is to provide a process for producing a glycoprotein having the human G-CSF activity.

The glycoprotein has a sugar chain portion and a polypeptide which is represented by all or part of the amino acid sequence shown below:

| Thr | Pro | Leu | Gly | Pro | Ala | Ser | Ser | Leu | Pro | Gln |
| Ser | Phe | Leu | Leu | Lys | Cys | Leu | Glu | Gln | Val | Arg |
| Lys | Ile | Gln | Gly | Asp | Gly | Ala | Ala | Leu | Gln | Glu |
| Lys | Leu | (Val | Ser | Glu)$_m$ | Cys | Ala | Thr | Tyr | Lys | Leu |
| Cys | His | Pro | Glu | Glu | Leu | Val | Leu | Leu | Gly | His |
| Ser | Leu | Gly | Ile | Pro | Trp | Ala | Pro | Leu | Ser | Ser |
| Cys | Pro | Ser | Gln | Ala | Leu | Gln | Leu | Ala | Gly | Cys |
| Leu | Ser | Gln | Leu | His | Ser | Gly | Leu | Phe | Leu | Tyr |
| Gln | Gly | Leu | Leu | Gln | Ala | Leu | Glu | Gly | Ile | Ser |
| Pro | Glu | Leu | Gly | Pro | Thr | Leu | Asp | Thr | Leu | Gln |
| Leu | Asp | Val | Ala | Asp | Phe | Ala | Thr | Thr | Ile | Trp |
| Gln | Gln | Met | Glu | Glu | Leu | Gly | Met | Ala | Pro | Ala |
| Leu | Gln | Pro | Thr | Gln | Gly | Ala | Met | Pro | Ala | Phe |
| Ala | Ser | Ala | Phe | Gln | Arg | Arg | Ala | Gly | Gly | Val |
| Leu | Val | Ala | Ser | His | Leu | Gln | Ser | Phe | Leu | Glu |
| Val | Ser | Tyr | Arg | Val | Leu | Arg | His | Leu | Ala | Gln |
| Pro | | | | | | | | | | |

(where m is 0 or 1).

Fig. 1 shows the sequences of three different probes, IWQ, A and LC;

Fig. 2 shows the nucleotide sequence of a pHCS-1 insert;

Fig. 3 shows the nucleotide sequence of a cDNA insert in pBRG4;

Fig. 4 shows the nucleotide sequence of a cDNA insert in pBRV2;

Fig. 5 shows the nucleotide sequence of a human chromosomal gene coding for human G-CSF;

Fig. 6 shows the restriction enzyme cleavage sites of the human chromosomal gene coding for human G-CSF;

Fig. 7 shows schematically the structure of pMLCE3α;

Fig. 8 shows schematically the structure of pTNCE3α; and

Fig. 9 shows schematically the structures of pD26SVCE3α and pDRCE3α.

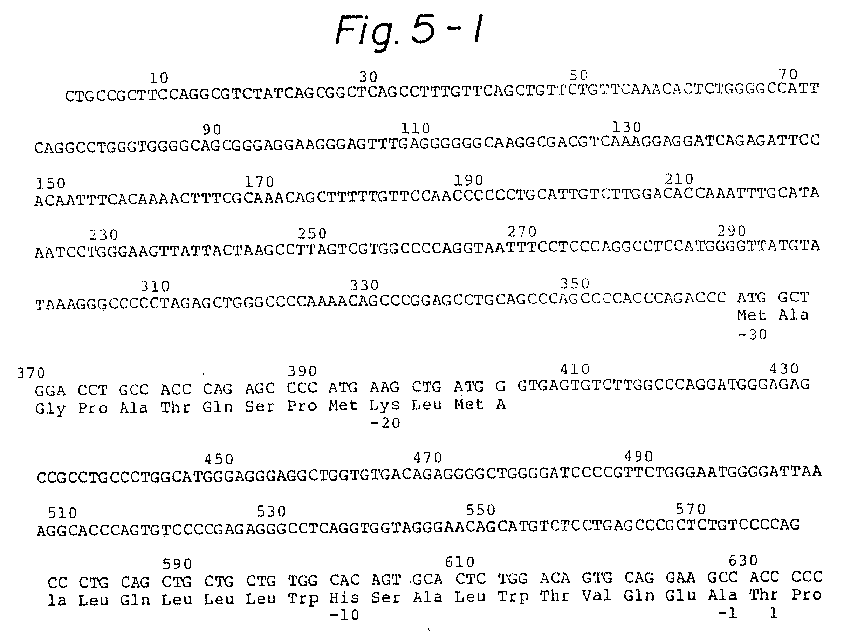

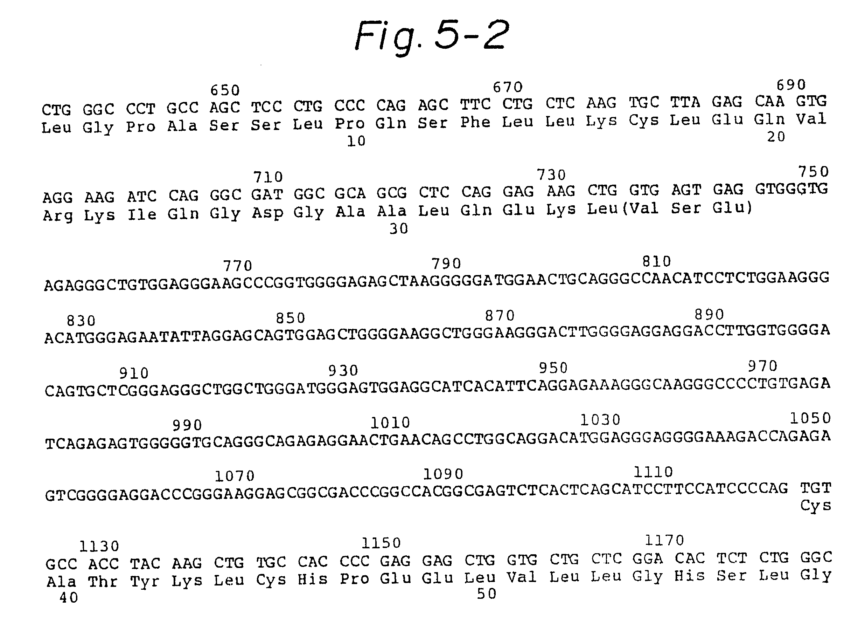

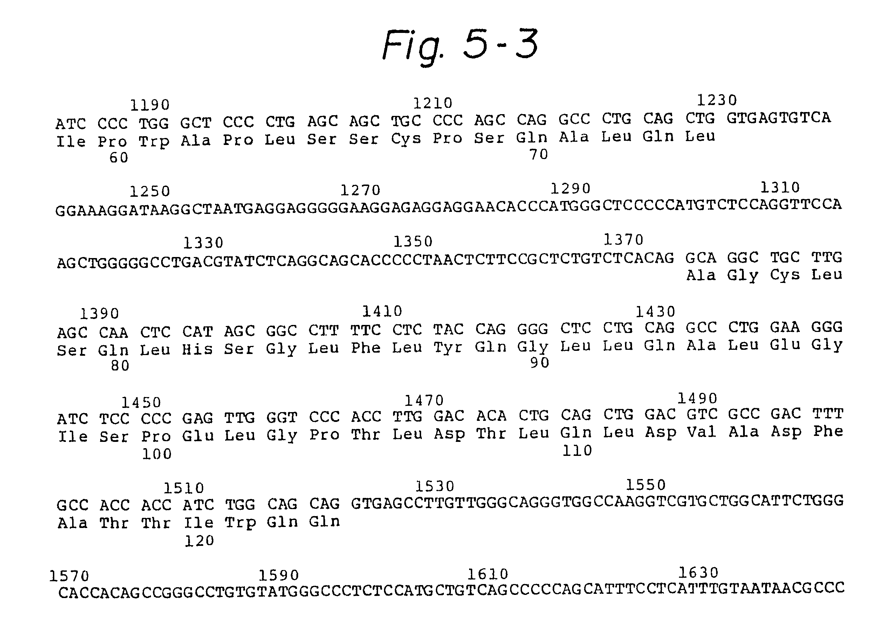

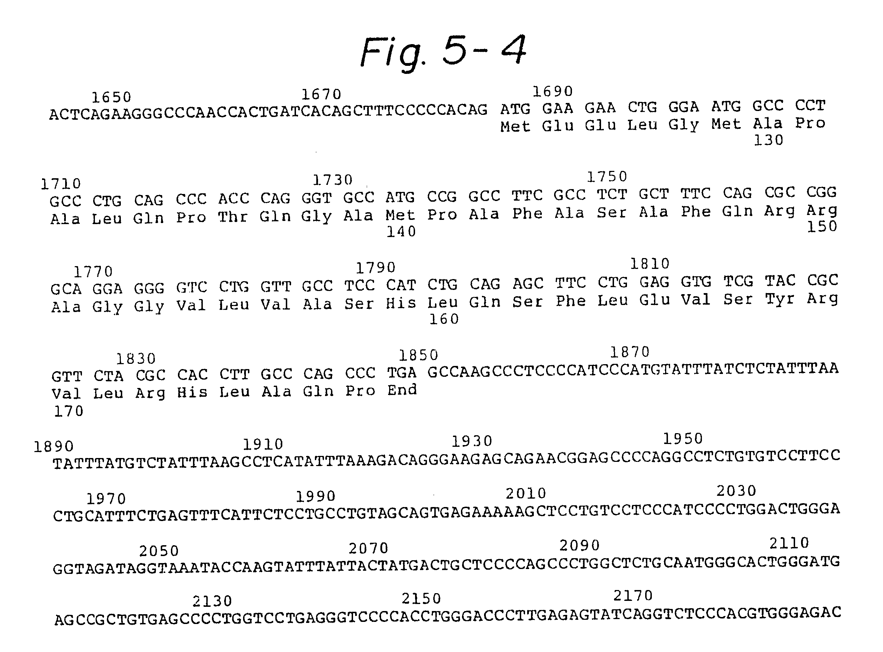

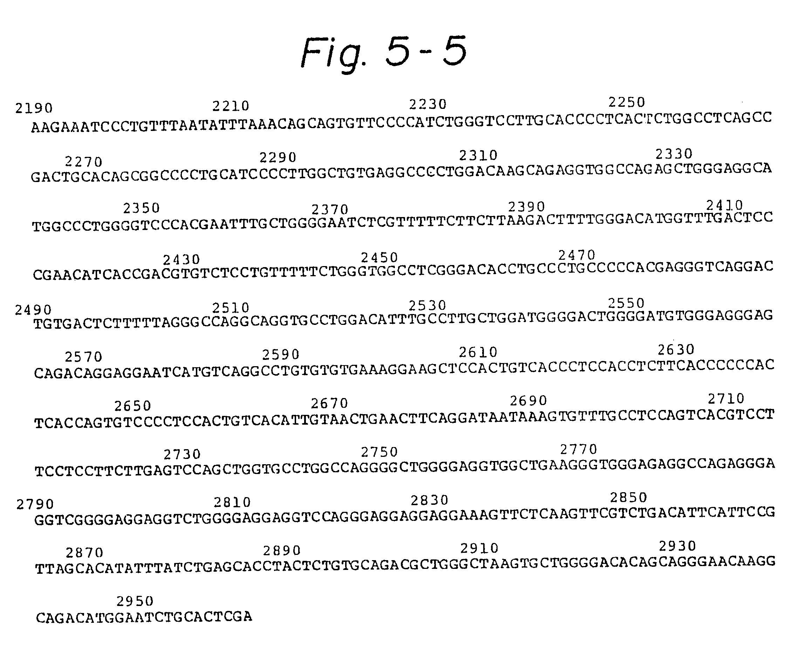

The human chromosomal gene of the present invention contains a nucleotide sequence that takes part in transcriptional control and it also contains all or part of the nucleotide sequence shown in Fig. 5.

A chromosomal gene may be obtained by first preparing from human cells a set of recombinants containing a human chromosomal gene (the set is hereunder referred to as a human chromosomal gene library), then subjecting said human chromosomal gene library to screening by known procedures.

The human chromosomal gene may be supplied from any type of human cells such as cells extracted from the liver or kidney or cultured cells such as tumor cells. A human chromosomal gene library may be prepared from human cells by any of the known methods [see Maniatis et al., Cell, 15, 687 (1978); and Maniatis et al., Molecular Cloning, Cold Spring Harbor Laboratory, p. 269 ff. (1982)], which are illustrated below:

extract a human chromosomal DNA from such sources as human fetal liver with phenol or other appropriate chemicals; digest the extracted DNA partially or completely with an appropriate restriction enzyme to obtain a DNA fragment of an appropriate length; insert the DNA fragment into a λ-phage vector DNA fragment with a T4 DNA ligase or other appropriate ligases, with a linker containing the restriction site for an appropriate enzyme such as EcoRI being optionally attached; subsequently, obtain λ-phage particles by in vitro packaging method and transform host cells such as E. coli with the resulting λ-phage particles.

Examples of the λ-phage usable as the vector in the above procedures include Charon 4A and EMBL-3 and EMBL-4.

Screening for the phage harboring the desired gene from a human chromosomal gene library may be performed by, for example, the following procedures.

A human G-CSF protein obtained from human G-CSF producing cells is purified and a partial amino acid sequence of the protein is determined. In a separate step, messenger RNA (mRNA) is extracted from the G-CSF producing cells and a complementary DNA to the mRNA (cDNA) is synthesized. A group of recombinant DNAs which contain the thus obtained cDNA (cNDA library) is thereafter prepared. Screening of the cDNA library is conducted using an olygonucleotide probe which has been chemically synthesized on the basis of the aforementioned amino acid sequence (see "Molecular Cloning", ibid.)

A DNA fragment which has all or part of the full-length DNA encoding a human G-CSF polypeptide is obtained from the cDNA clones. The human chromosomal gene library already described in this specification is screened by the plaque hybridization method with this DNA fragment being used as a probe [Benton and Davis, Science, 196, 180 (1977)].

The fragment harboring the thus cloned chromosomal gene coding for the polypeptide having the human G-CSF activity may be re-inserted in an appropriate vector DNA for the purpose of transforming other eukaryotic host cells. By introducing an appropriate promoter and an expression-associated sequence into the vector, the gene can be expressed in an individual host cell.

Illustrative host cells derived from mammalian cells include COS cells, Chinese hamster ovary (CHO) cells, C-127 cells and Hela cells. An illustrative vector that may be used to transform these cells is pdBPV-1 [see Sarver et al.; Proc. Natl. Acad. Sci., USA, 79, 7147 (1982)]. The vectors used to transform these cells contain origin, selection marker, a promoter preceding in position the gene to be expressed, RNA splicing site, polyadenylation signal, etc.

Illustrative promoters that may be used for gene expression in mammalian cells include the promoters of a retrovirus, polyoma virus, adenovirus, simian virus 40 (SV40), etc. If the promoter of SV40 is used, the desired gene expression may be readily achieved in accordance with the method of Mulligan et al. described in Nature, 277, 108 (1979).

Illustrative origins that can be used include those derived from SV40, polyoma virus, adenovirus, bovine papilloma virus (BPV), etc. Illustrative selection markers that can be used include the phosphotransferase APH (3') II or I (neo) gene, thymidine kinase (TK) gene, E. coli xanthineguanine phosphoribosyltransferase (Ecogpt) gene, dihydrofolate reductase (DHFR) gene, etc.

In order to obtain polypeptides having the human G-CSF activity from the above listed host-vector systems, the following procedures may be used: the gene coding for the peptide having the human G-CSF activity is inserted at a suitable site in one of the vectors mentioned above; the host cell is transformed with the resulting recombinant DNA; and the obtained transformants are cultured. The desired polypeptide may be isolated and purified from the cell or culture solution by any one of the known techniques.

Eukaryotic genes are generally held to exhibit polymorphysm as is known for the case of the human interferon gene [see Nishi et al., J. Biochem., 97, 153 (1985)] and this phenomenon may cause substitution of one or more amino acids or a change in the nucleotide sequence but no change in the amino acid sequence at all.

It is also known that each allele of a chromosome may have a different nucleotide sequence [see, for example, Weissmann et al., Phil. Trans. R. Soc. Lond. B299, 7 (1982)].

The G-CSF activity may also be possessed by a polypeptide which is deficient of one or more of the amino acids in the amino acid sequence or which has such amino acids added thereto, or a polypeptide which has one or more of these amino acids replaced by one or more amino acids. It is also known that a polypeptide obtained by converting each of the cysteine codons in the human interleukin-2 (IL-2) gene to a serine codon has the activity of interleukin-2 [Wang et al., Science, 224, 1431 (1984)]. Therefore, so long as the polypeptides, either naturally occurring or chemically synthesized, have the human G-CSF activity, all of the genes that code for these polypeptides, recombinant vectors containing these genes, transformants obtained by such recombinant vectors, and the polypeptides or glycoproteins that are obtained by cultivating such transformants are included within the scope of the present invention.

Hereunder outlined are the processes for producing the chromosomal gene of the present invention coding for a polypeptide having the human G-CSF activity, a recombinant vector having said gene and a transformant having this recombinant vector, and a process for producing a glycoprotein having the human G-CSF activity.

(1) Probe preparation

A homogeneous human CSF protein was purified from the supernatant of a culture of a tumor cell line, CHU-2 (deposited at Collection Nationale de Cultures de Microorganismes, or C.N.C.M., under Accession Number I-483), and its amino acid sequence from the N terminus was determined. Fragments were obtained by decomposition with bromocyan and treatment with trypsin and the amino sequences of these

4

fragments were also determined [Example 3(i), (ii) and (iii)].

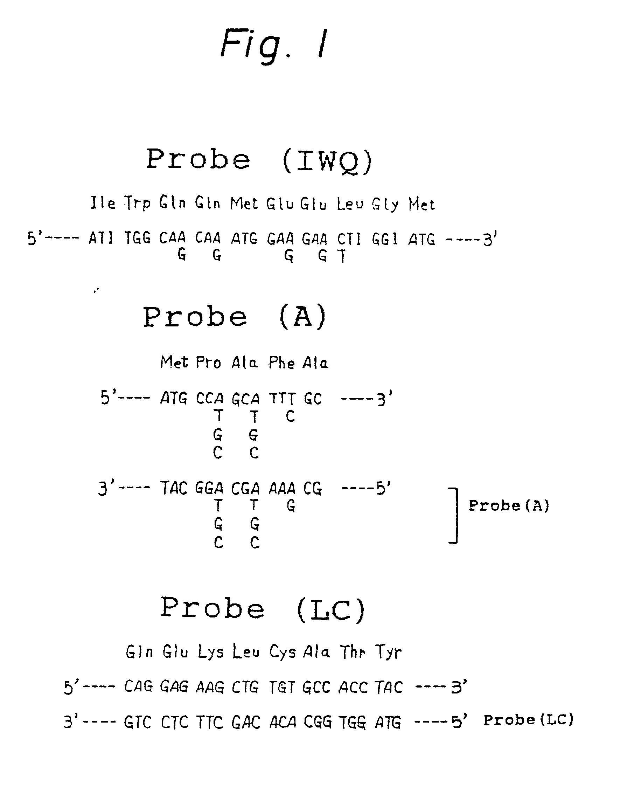

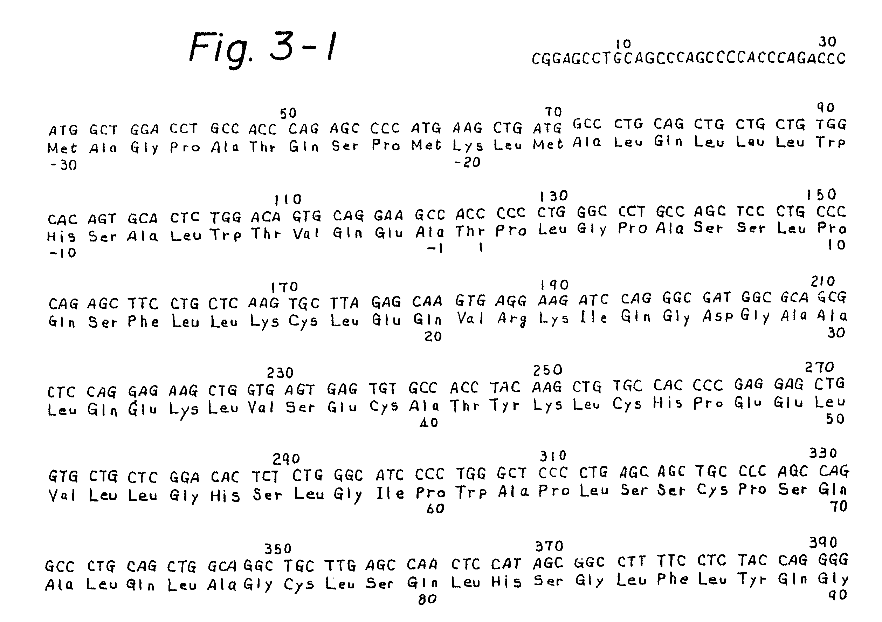

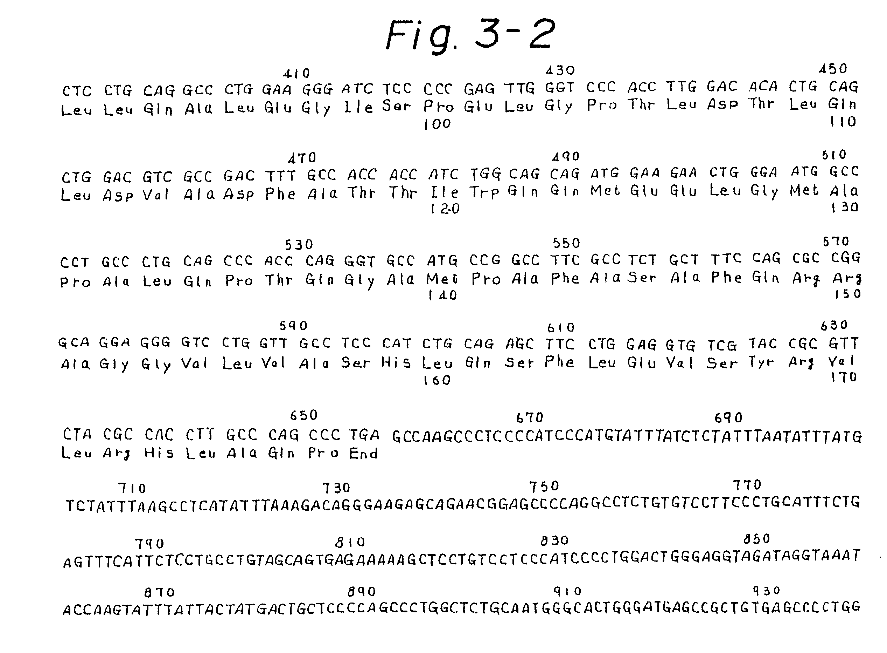

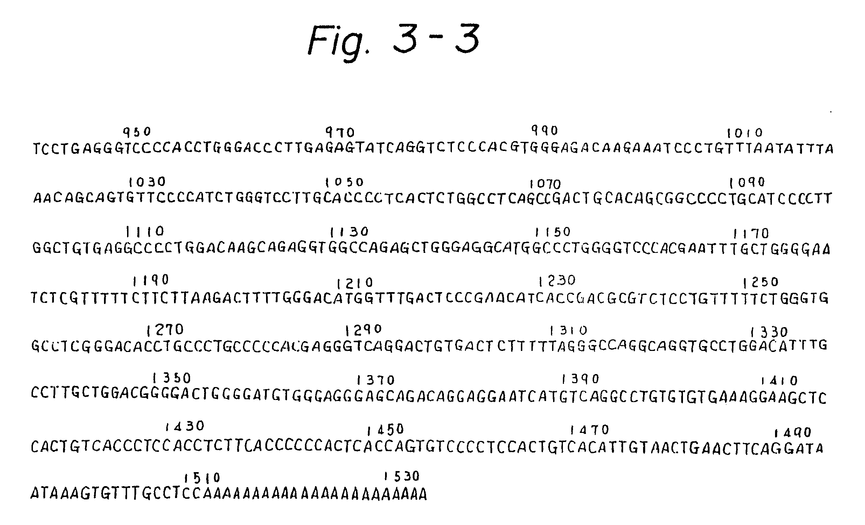

From the determined amino acid sequences, three nucleotide probes, (A), (LC) and (IWQ), having the sequences shown in Fig. 1 were synthesized (Example 4). Probe (A) was of the mixed type composed of 14 successive nucleotides. Probe (IWQ) was composed of 30 successive nucleotides with deoxyinosine and was a probe of the type used in the cloning of the human cholecystokinin gene [Takahashi et al., Proc. Natl. Acad. Sci., USA, 82, 1931 (1985)]. Probe (LC) was a 24-nucleotide probe that was synthesized from the nucleotides at 32 - 39 positions from the N terminus of the amino acid sequence shown in Example 3(i) on the basis of nucleotide sequence shown in Fig. 3.

Chemical synthesis of nucleotides can be achieved by applying the improved phosphotriester method to the solid phase method and has been reviewed by Narang [Tetrahedron, 39, 3-22 (1983)]. These nucleotides can also be synthesized using an automatic DNA synthesizer (Applied Biosystems).

Probes based on amino acid sequences at positions other than those in the above-mentioned probes may also be used.

(2) Construction of cDNA library

CHU-2 cells were homogenized after addition of a guanidine thiocyanate solution and the total RNA was obtained by CsCl density gradient centrifugation.

Poly(A$^+$) RNA was isolated from the total RNA by column chromatography on oligo(dT)-cellulose. Thereafter, a single-stranded cDNA was synthesized with a reverse transcriptase, and RNase H and E. coli DNA polymerase I were added to obtain a double-stranded cDNA. A dC chain was attached to the obtained double-stranded cDNA, which was joined to a vector, pBR322, to which a dG chain had been attached at the Pst I cleavage site. The resulting recombinant DNA was used to transform a strain of E. coli, X1776, and a pBR322-line cDNA library was constructed (Examples 5 and 6).

In a similar manner, the double-stranded cDNA was joined to the λgt10 vector with the EcoRI linker and λ-phage line cDNA library was constructed (Example 7).

(3) Screening

Recombinants derived from the pBR322-line cDNA library were fixed on Whatmann 541 filter paper and a single clone could be selected by colony hybridization with $^{32}$P-labelled probe (IWQ). Further study with the Southern blotting method [Southern, J. Mol. Biol., 98, 503 (1975)] showed that this clone also hybridized with probe (A). The nucleotide sequence of this clone was determined by the dideoxy method [Sanger, Science, 214, 1205 (1981)].

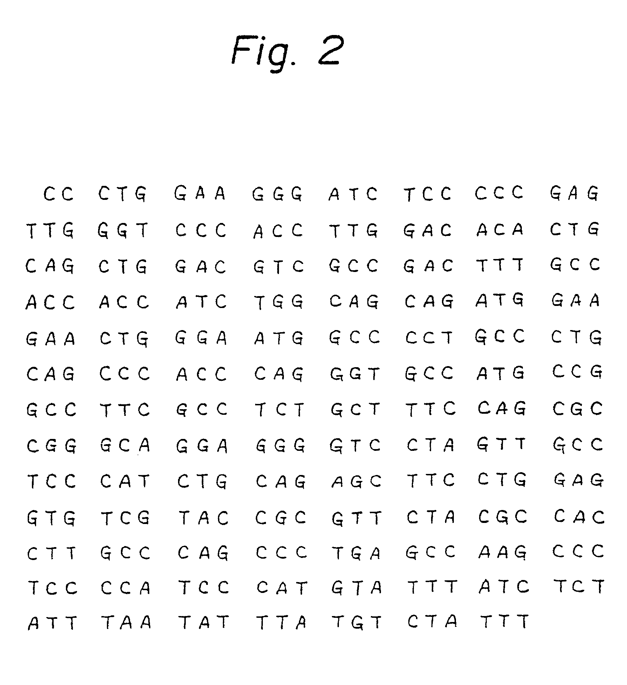

The nucleotide sequence of the obtained cDNA insert is shown in Fig. 2, from which one can see that this insert consisted of 308 base pairs including probes (IWQ) and (A), and had an open reading frame coding for 83 amino acids containing the amino acid sequence shown in Example 3(iii). The pBR322-derived plasmid containing these 308 base pairs is hereunder referred to as pHCS-1 (Example 8).

A DNA fragment containing the 308 base pairs obtained from pHCS-1 was radiolabelled by the nick translation method (see Molecular Cloning, ibid.) and, with this fragment used as a probe, the λgt10-derived cDNA library was screened by plaque hybridization [Benton and Davis, Science, 196, 180 (1977)] to obtain five clones. The nucleotde sequence of a clone which was believed to contain cDNA was determined by the same method as described above (Fig. 3).

As shown in Fig. 3, this cDNA insert had a single large open reading frame.

The amino acid sequence encoded by this cDNA can be deduced as shown in Fig. 3.

Escherichia coli strain X1776 harboring pBR322 which had this cDNA at the EcoRI cleavage site has been deposited with the Fermentation Research Institute, the Agency of Industrial Science and Technology (FERM BP-954).

This cDNA was joined to pBR327 [Soberon et al., Gene, 9, 287 (1980)] at the EcoRI site and the resulting plasmid is hereunder referred to as pBRG4.

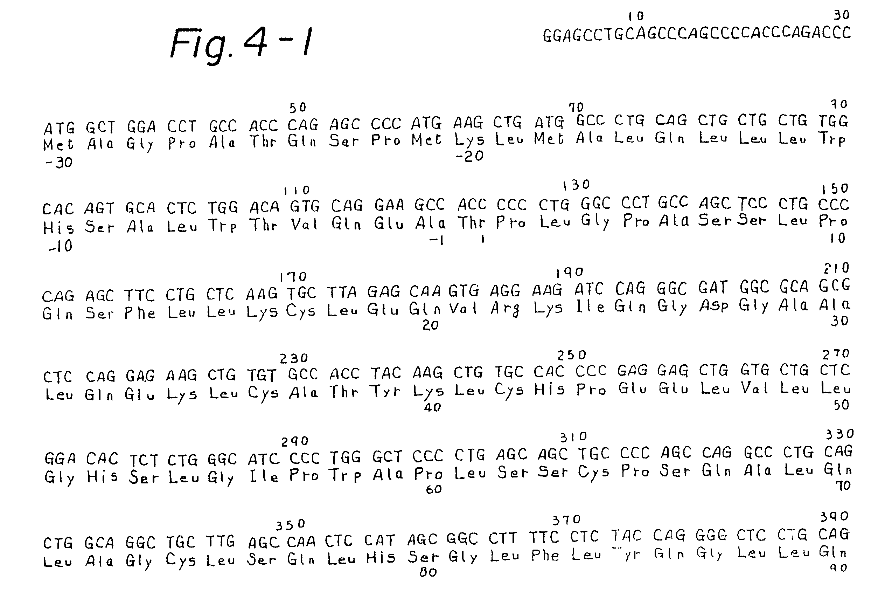

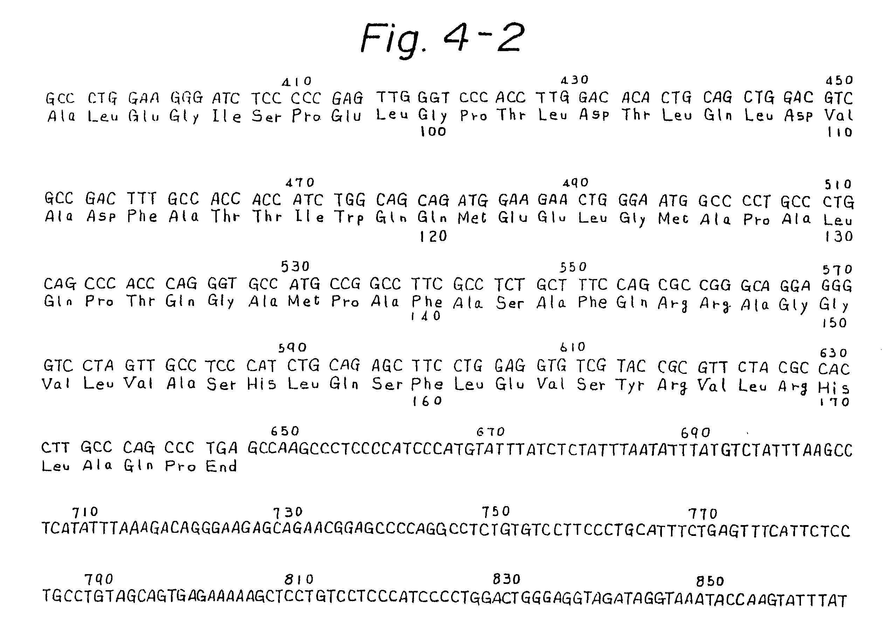

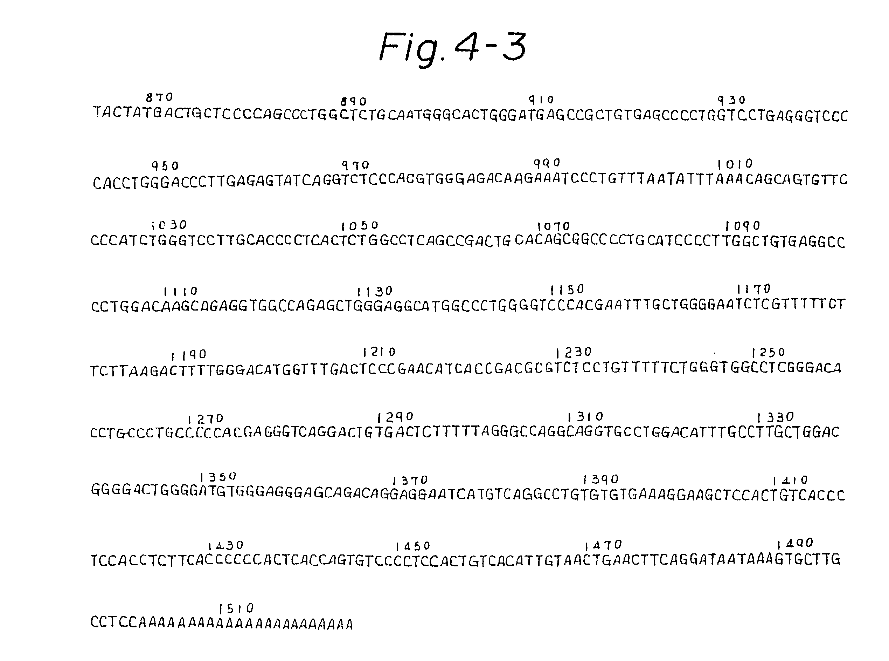

The thus obtained pBRG4 was treated with a restriction enzyme, EcoRI, to obtain a DNA fragment containing cDNA of about 1500 base pairs. This fragment was radiolabelled by the nick translation method (see Molecular Cloning, ibid.) and, with this radiolabelled DNA fragment being used as a probe, the λgt10-derived cDNA library was screened once again by plaque hybridization (see Benton and Davis, ibid.) In this plaque hybridization, two sheets of λ-phage DNA fixed nitrocellulose filter paper were prepared; one of these sheets was used for the above-mentioned plaque hybridization and another one was subjected to plaque hybridization with the already described probe (LC). The phages which turned positive for both probes were selected. A clone which has a "full-length" cDNA was selected and the nucleotide sequence of

EP 0 220 520 B1

the cDNA insert as determined by the dideoxy method is shown in Fig. 4.

This cDNA had a single large open reading frame and the amino acid sequence that would be encoded by this cDNA was deduced as shown in Fig. 4 (Example 10).

Escherichia coli strain X1776 harboring pBR322 which had this cDNA at the EcoRI cleavage site has been deposited with the Fermentation Research Institute, the Agency of Industrial Science and Technology (FERM BP-955).

This cDNA was joined to pBR327 at the EcoRI site to form a plasmid which is hereunder referred to as pBRV2.

(4) Screening a human chromosomal gene library

A human chromosomal gene library that was prepared in accordance with the procedures described by Maniatis et al. (Molecular Cloning, ibid.) was subjected to screening with the pHCS-1 shown above. Probes that may be employed in screening include: a pHCS-1-derived 308-bp DNA fragment, a pBRG4-derived ca. 1500-bp DNA fragment, a pBRV2-derived ca. 1500-bp DNA fragment, a DNA fragment of an appropriate length containing part of one or more of these DNA fragments, as well as the aforementioned oligonucleotide probes [i.e., (IWQ), (A) and (IC)]. The case of using the pHCS-1 DNA fragment is hereunder described.

This DNA fragment was radiolabelled with $^{32}$P in accordance with the nick translation method [see Roop et al., Cell, 15, 431 (1978)]. With the resulting $^{32}$P-labelled fragment used as a probe, the human chromosomal gene library was subjected to screening by plaque hydridization (see Benton and Davis, ibid.) so as to obtain ten-odd clones.

After recovering DNA from the clones, a restriction enzyme map was prepared by known procedures [Fritsch et al., Cell, 19, 959 (1980)].

With the same DNA probe being used, Southern blotting (see Southern, ibid.) was conducted and it was found that a DNA fragment of about 4 kb that was cut out with EcoRI and XhoI could potentially contain a region for encoding the human G-CSF polypeptide. Therefore, the ca. 4-kb DNA fragment was inserted into pBR327 at the EcoRI site using an EcoRI linker so as to obtain pBRCE3$\beta$. With this plasmid being used as a base sequencing DNA, the nucleotide sequence of the ca. 3-kb portion of that ca. 4-kb DNA fragment was determined by the dideoxy method. As a result, said DNA fragment was found to be a gene coding for the human G-CSF polypeptide (Fig. 5).

E. coli strain X1776 harboring pBRCE3$\beta$ (i.e. the plasmid pBR327 having said ca. 4-kb DNA fragment inserted into the EcoRI site) has been deposited with the Fermentation Research Institute, the Agency of Industrial Science and Technology (FERM BP-956).

Comparison between the pBRG4 cDNA insert shown in Fig. 3 and the pBRV2 cDNA insert shown in Fig. 4 revealed that the DNA fragment under discussion contained five exon portions and that it coded for the amino acid sequences deduced from pBRG4 and pBRV2.

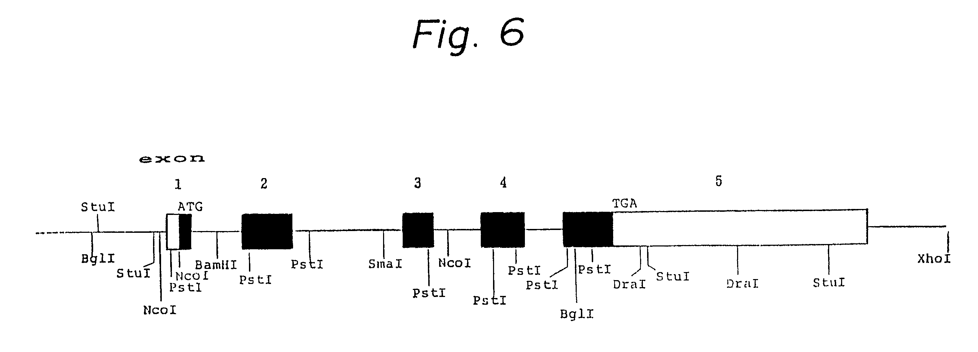

Fig. 6 shows the restriction enzyme cleavage sites of the obtained gene.

This DNA fragment contained the chromosomal gene of human G-CSF, or the preceding region to be transcribed to human G-CSF mRNA, plus a nucleotide sequence taking part in transcriptional control [Benoist and Chambon, Nature, 290, 304 (1981); and Breathnack and Chambon, Ann. Rev. Biochem., 50, 349 (1981)].

(5) Construction of recombinant vectors containing the Chromosomal gene

An expression vector for COS cells was constructed by the following procedures.

The plasmid pBRCE3$\beta$ that was obtained in (4) and which contained the chromosomal gene shown in Fig. 5 was treated with EcoRI.

The pSVH$^+$K$^+$ plasmid described by Banerji et al. in Cell, 27, 299 (1981) was treated with KpnI to remove the globin gene. The plasmid was further subjected to partial digestion with HindIII so as to remove part of the late gene of SV40. The fragments were re-joined to prepare an expression vector pML-E$^+$.

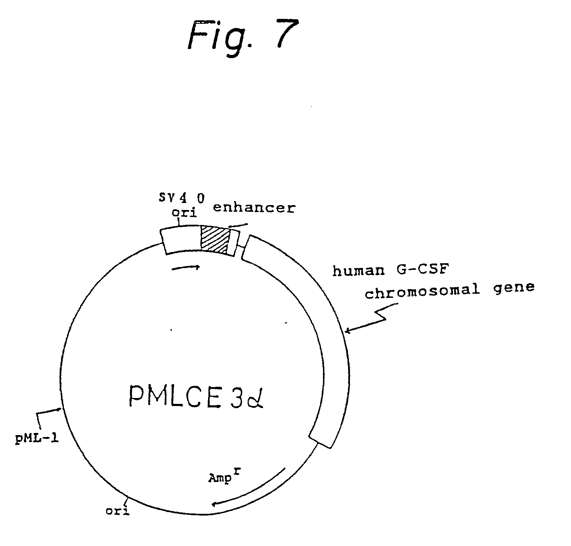

This vector was treated with the restriction enzyme, EcoRI, and dephosphorylated with an alkaline phosphatase (Takara Shuzo Co., Ltd.) to obtain a vector DNA, which was linked to the aforementioned chromosomal DNA fragment with the aid of a T4DNA ligase (Takara Shuzo Co., Ltd.) to obtain pMLCE3$\alpha$ which was a recombinant vector for COS cells (Example 12). As shown in Fig. 7, this plasmid contained the enhancer of SV40 gene, the replication origin of SV4O, the replication origin of pBR322 and the pBR322-derived $\beta$-lactamase gene (Amp$^r$), and had the human G-CSF chromosomal gene joined downstream from the enhancer of SV40 gene.

6

An expression vector for C127 cells was constructed by the following procedures. A DNA fragment containing the chromosomal CSF gene was cut out with an appropriate restriction enzyme from pMLCE3α which was the expression vector for COS cells. This fragment was joined, with a T4DNA ligase, to a DNA fragment containing the origin of bovine papilloma virus (BPV) and a DNA fragment containing the early promoter of SV40. The resulting pTNCE3α was an expression vector that had a chromosomal CSF gene linked downstream from the early promoter of SV40 and which contained a 65% portion of BPV (Example 16).

The expression vector for CHO cells had two DNA fragments linked together by a T4DNA ligase; one fragment contained the chromosomal CSF gene and the early promoter of SV40 as in the case of the expression vector for C127 cells, and the other fragment contained a pAdD26SVpA-derived dhfr gene. The resulting pD26SVCE3α was an expression vector that had the chromosomal CSF gene downstream of the SV40 promoter and, the dhfr gene downstream of the principal late promoter of adenovirus (Example 18).

### (6) Expression in animal cells

A representative example using COS cells as host cells is hereunder described and, for further details, see the relevant working examples.

COS cells, which were derived from monkey CV-1 cells and which had been transformed by SV40-origin deficient mutant to express the large-size T antigen of SV40 [see Gluzman et al., Cell, 32, 175 (1981)-], were transformed by the vector pMLCE3α which was obtained in (5) and which contained the human chromosomal G-CSF gene. The supernatant of the culture of the COS cells showed the human G-CSF activity (Example 13).

The COS cells were recovered and subjected to mRNA analysis, which showed the existence of two mRNAs that corresponded to the amino acid sequences depicted in Fig. 3 and Fig. 4, respectively (Example 14).

### Examples

Before the present invention is described in greater detail with reference to working examples, the following referential example is provided for the purpose of illustrating the methods of assaying the CSF activity.

### Referential Example: Assaying CSF Activity

The following methods were used to determine the CSF activity (hereunder abbreviated as CSA) in the present invention.

### CSA assay

(a) With human bone marrow cells:

Single-layer soft agar cultivation was conducted in accordance with the method of Bradley, T.R. and Metcalf, D. (Aust. J. Exp. Biol. Med. Sci., 44, 287-300, 1966). More specifically, 0.2 ml of a bovine fetal serum, 0.1 ml of the sample, 0.1 ml of a human bone marrow nonadherent cell suspension (1 - 2 x $10^5$ nuclear cells), 0.2 ml of a modified McCoy's 5A culture solution, and 0.4 ml of a modified McCoy's 5A culture solution containing 0.75% of agar were mixed, poured into a plastic dish for tissue culture (35 mm$^\emptyset$), coagulated, and cultured at 37°C in 5% $CO_2$/95% air and at 100% humidity. Ten days later, the number of colonies formed was counted (one colony consisting of at least 50 cells) and CSA was determined with one unit being the activity required for forming one colony.

(b) With mouse bone marrow cells:

A horse serum (0.4 ml), 0.1 ml of the sample, 0.1 ml of a C3H/He (female) mouse bone marrow cell suspension (0.5 - 1 x $10^5$ nuclear cells), and 0.4 ml of a modified McCoy's 5A culture solution containing 0.75% of agar were mixed, poured into a plastic dish for tissue culture (35 mm$^\emptyset$), coagulated, and cultured for 5 days at 37°C in 5% $CO_2$/95% air and at 100% humidity. The number of colonies formed was counted (one colony consisting of at least 50 cells) and CSA was determined with one unit being the activity for forming one colony.

The modified McCoy's 5A culture solution used in each of the methods (a) and (b) and the human bone marrow nonadherent cell suspension used in (a) were prepared by the following procedures.

Modified McCoy's 5A culture solution (double concentration)

Twelve grams of McCoy's 5A culture solution (Gibco), 2.55 g of MEM amino acid-vitamin medium (Nissui Seiyaku Co., Ltd.), 2.18 g of sodium bicarbonate and 50,000 units of potassium penicillin G were dissolved twice in 500 ml of distilled water and the solution was aseptically filtered through a Millipore filter (0.22 μm).

Human bone marrow nonadherent cell suspension

A bone marrow fluid obtained from a healthy person by sternal puncture was diluted 5-fold with an RPMI 1640 culture solution, plated over a Ficoll-Paque solution (Pharmacia Fine Chemicals) and centrifuged at 400 x g for 30 minutes at 25°C. The interfacial cell layer (specific gravity <1.077) was recovered. The cells were washed, adjusted to a concentration of $5 \times 10^6$ cells/ml with an RPMI 1640 culture solution containing 20% of bovine fetal serum, poured into a 25-cm$^2$ plastic flask for tissue culture, and incubated for 30 minutes in a $CO_2$ incubator. Nonadherent cells were recovered in the supernatant, poured into a plastic flask (25 cm$^2$) and incubated for 2 hours and a half. Nonadherent cells in the supernatant were collected and used in an assay.

Example 1: Establishment of CHU-2

A tumor of a patient with oral cavity cancer wherein pronounced increase was observed in the number of neutrophiles was transplanted into nu/nu mice. About 10 days after the transplantation, the increase in the weight of the tumor and in the number of neutrophiles was significant. Twelve days after the transplantation, the tumor was extracted aseptically, dissected into cubes of 1 - 2 mm$^3$ and cultured in the following manner.

Ten to fifteen cubes of the tumor were put into a 50-ml plastic centrifugal tube. After addition of 5 ml of a trypsin solution (containing 0.25% of trypsin and 0.02% of EDTA), the tube was shaken for 10 minutes in a warm bath at 37°C and the supernatant was discarded. Another 5 ml of the same trypsin solution was added and trypsin digestion was conducted under agitation for 15 minutes at 37°C. The supernatant cell suspension was recovered and stored in ice after the trypsin had been inactivated by addition of 1 ml of a bovine fetal serum.

After repeating these procedures once again, the cell suspension was recovered, combined with the previously obtained suspension, and centrifuged at 400 x g for 10 minutes to obtain a cell pellet. The pellet was washed twice with F-10 containing 10% of a bovine fetal serum and was thereafter loaded in a plastic culture flask (25 cm$^2$) to give a cell concentration of $5 \times 10^6$ cells/flask. After incubation overnight in a $CO_2$ incubator (5% $CO_2$ and 100% humidity) with an F-10 culture solution containing 10% of a bovine fetal serum, the supernatant was removed together with the nonadherent cells, and culture was continued with a fresh supply of culture solution. Six days after the start of culture, the flask became full of the cells and the culture solution was replaced by a fresh one. On the next day, the culture solution was discarded and the flask was charged with 2 ml of an anti-mouse erythrocyte antibody (Cappel) diluted 5-fold with RPMI 1640 and 2 ml of a guinea pig complement (Kyokuto Seiyaku Co., Ltd.) diluted 2.5-fold with RPMI 1640. After incubation for 20 minutes at 37°C, the culture was washed twice with F-10 containing 10% of a bovine fetal serum and the nu/nu mouse derived fibroblasts were removed. Subsequently, an F-10 culture solution containing 10% of a bovine fetal serum was added and cultivation was conducted for 2 more days. Thereafter, some of the cells were recovered and subjected to cloning by the limiting dilution method.

The resulting 11 clones were checked for their CSF activity and one clone (CHU-2) exhibited activity about 10 times as high as that of the other clones.

Example 2: Isolation of CSF

The cells established in Example 1 were grown in a completely dense population (grown to confluency) in two culture flasks (150 cm$^2$). The cells were recovered, suspended in 500 ml of an F-10 culture solution containing 10% of a bovine fetal serum, transferred into a glass roller bottle of 1580 cm$^2$ (Belco), and whirl-cultured at 0.5 rpm. When the cells were found to have grown in a completely dense population on the inner wall of the roller bottle, the culture solution was replaced by a serum-free RPMI 1640. After 4-day culture, the supernatant of the culture was recovered and cultivation was continued with F-10 containing 10% of a bovine fetal serum being added. After 3-day culture, the culture solution was again replaced by a serum-free RPMI 1640 and the supernatant of the culture was recovered 4 days later. By repeating these procedures, 500 ml of the serum-free supernatant of culture per bottle was obtained each week. In addition, this method enabled the supernatant of culture to be recovered, with the cells maintained over a significantly prolonged period.

A batch consisting of 5,000 ml of the supernatant of the culture obtained was mixed with 0.01% of Tween 20 and concentrated about 1000 times by ultrafiltration with Hollow Fiber DC-4 and Amicon PM-10 (Amicon). The concentrate was purified by the following steps.

(i) A portion (5 ml) of the concentrated supernatant of culture was subjected to gel filtration on an Ultrogel AcA54 column (4.6 cm$^\varnothing$ x 90 cm$^L$; LKB) at a flow rate of ca. 50 ml/hr with 0.01 M Tris-HCl buffer (pH 7.4) containing 0.15 M NaCl and 0.01% Tween 20 (Nakai Kagaku Co., Ltd.). The column had been calibrated with bovine serum albumin (Mw; 67,000), ovoalbumin (Mw; 45,000) and cytochrome C (Mw; 12,400). After completion of the gel filtration, 0.1 ml of each of the fractions was diluted 10-fold and screened for the active fractions by the above-described method of CSA assay (b). The fractions for Ve = 400 - 700 ml were found to exhibit macrophage-dominant CSA while the fractions for Ve = 800 - 1200 ml showed granulocyte-dominant CSA. Therefore, the latter fractions were collected and concentrated to a volume of ca. 5 ml on an ultrafiltration apparatus with PM-10 (Amicon).

(ii) To the cocentrated fractions was added an aqueous solution of 0.1% trifluoroacetic acid containing 30% of n-propanol (for determination of amino acid sequence; available from Tokyo Kasei K.K.) After the mixture had been left to stand in ice for about 15 minutes, the precipitate was removed by centrifugation for 10 minutes at 27,000 x g. The supernatant was adsorbed on a $\mu$-Bondapak C18 column (8 mm x 30 cm for semipreparatory use; Waters) equilibrated with the aqueous solution containing n-propanol and trifluoroacetic acid; the column was continuously eluted with an aqueous solution of 0.1% trifluoroacetic acid which contained n-propanol having a linear concentration gradient of 30 - 60%. A high performance liquid chromatographic apparatus, Hitachi Model 685-50 (Hitachi, Ltd.), and a detector, Hitachi Model 638-41 (Hitachi, Ltd.) were employed to determine the absorptions at 220 nm and 280 nm simultaneously. After elution, 10 $\mu$l of each of the fractions was diluted 100-fold and screened for the active fractions by the above-described method of CSA assay (b). The peaks eluted with 40% n-propanol were found to have CSF activity, so they were collected, re-chromatographed under the same conditions, and assayed for CSA by the same method. Again, CSF activity was observed in the peaks at 40% n-propanol. Therefore, these peaks were collected (4 fractions = 4 ml) and freeze-dried.

(iii) The freeze-dried powder was dissolved in 200 $\mu$l of an aqueous solution of 0.1% trifluoroacetic acid containing 40% of n-propanol, and the solution was subjected to high performance liquid chromotography on TSK-G 3000SW column (Toyo Soda Manufacturing Co., Ltd.; 7.5 mm x 60 cm). Elution was conducted with the same aqueous solution at a flow rate of 0.4 ml/min and the fractions were taken in 0.4-ml portions with a fraction collector, FRAC-100 (Pharmacia Fine Chemicals). Each of the fractions taken was checked for its CSA by the same method as described above and activity was observed in the fractions for retention times of 37 - 38 minutes (ccrresponding to MW of ca. $2 \times 10^4$). The active factions were recovered and purified on an analytical $\mu$-Bondapak C18 column (4.6 mm x 30 cm). The main peaks were recovered and freeze-dried. The sample obtained was assayed by the method of CSA assay (a); it was found to have human G-CSF activity.

Example 3: Determination of Amino Acid Sequence

(i) Determination of N-terminal amino acid sequence

The sample was subjected to Edman degradation with a gas-phase sequencer (Applied Biosystems) and the resulting PTH amino acid was analyzed by routine procedures with a high performance liquid chromatographic apparatus (Beckman Instruments) and Ultrasphere-ODS column (Beckman Instruments). The column (5 $\mu$m; 4.6 mm$^\varnothing$ x 250 mm$^L$) was equilibrated with a starting buffer [aq. sol. containing 15 mM sodium acetate buffer (pH 4.5) and 40% acetonitrile] and injected with the sample (as dissolved in 20 $\mu$l of the starting buffer). Separation was effected by isocratic elution with the starting buffer. The flow rate was 1.4 ml/min and the column temperature was held at 40°C. Detection of the PTH amino acid was achieved utilizing the absorptions in the UV range at 269 nm and 320 nm. standard samples of PTH amino acid (Sigma) in 2-nmol portions were separated on the same line to determine their retention times, which were compared with those of the sample to be tested. As a result, the sample was found to have the following amino acid sequence of the 40 residues from N-terminus:

```
H₂N - Thr - Pro - Leu - Gly - Pro - Ala - Ser - Ser -
         (10)
Leu - Pro - Gln - Ser - Phe - Leu - Leu - Lys - Cys -
              (20)
Leu - Glu - Gln - Val - Arg - Lys - Ile - Gln - Gly
                    (30)
Asp - Gly - Ala - Ala - Leu - Gln - Glu - Lys - Leu -
                          (40)
Cys - Ala - Thr - Tyr - Lys -
```

(ii) Degradation with bromocyan

The sample was dissolved in 70% formic acid. To the solution, 200 equivalent amounts of bromocyan that had been purified by sublimation was added. The mixture was left overnight at 37°C for reaction. The reaction product was freeze-dried and fractionated by HPLC on a TSK G3000SW column (Toyo Soda Manufacturing Co., Ltd.) to obtain four peaks. The peaks were named CN-1, CN-2, CN-3 and CN-4 in the decreasing order of the molecular weight. The first two peaks (CN-1 and CN-2) had better yields and their amino acid sequences were analyzed with an automatic gas-phase sequencer (Applied Biosystems) under the same conditions as used in (i).

As a result, CN-1 was found to be a peptide from the N-terminus of G-CSF protein, and CN-2 had the followng amino acid sequence:

```
Pro - Ala - Phe - Ala - Ser - Ala - Phe -

Gln - Arg - Arg - Ala - Gly - Gly - Val -

Leu - Val - Ala - Ser - His - Leu - Gln -
```

(iii) Digestion with trypsin

The sample was dissolved in 0.1 M Tris-HCl buffer (pH 7.4) containing 8 M urea and the solution was mixed with 0.1 M Tris-HCl buffer (pH 7.4) containing 0.1% 2-mercaptoethanol to provide a final urea concentration of 2 M. A TPCK-treated trypsin (Sigma) was added such that the sample-to-enzyme ratio was 50:1. The mixture was held for 4 hours at 25°C and, after addition of an equal amount of TPCK-treated trypsin, the mixture was held for an additional 16 hours at 25°C. Thereafter, the reaction product was subjected to high-speed reverse-phased column chromatography on C8 column (Yamamura Kagaku K.K.), with elution conducted with 0.1% TFA containing n-propanol having a linear density gradient of 5 - 60%. While several peaks were obtained by measuring the absorption at 280 nm, the main peak was analyzed for its amino acid sequence with an automatic gas-phase sequencer (Applied Biosystems) under the same conditions as used in (i). As a result, the main peak was found to be a peptide having the following sequence which contained part of the CN-2 fragment shown in (ii):

```
Gln - Leu - Asp - Val - Ala - Asp - Phe - Ala - Thr -

Thr - Ile - Trp - Gln - Gln - Met - Glu - Glu - Leu -

Gly - Met - Ala - Pro - Ala - Leu - Gln - Pro - Thr -

Gln - Gly - Ala - Met - Pro - Ala - Phe - Ala - Ser -
```

Example 4: Preparation of DNA Probe

(i) Synthesis of probe (IWQ)

Thirty successive nucleotides (see Fig. 1) were prepared on the basis of the sequence of 10 amino acids (Ile-Trp-Gln-Gln-Met-Glu-Glu-Leu-Gly-Met) included within the amino acid sequence obtained in Example 3(iii). It will be necessary to make one comment about the notation of nucleotides shown in Fig. 1; for example, the nucleotide at 9-position from 5'-terminus is an equimolar mixture of dA and dG. The starting nucleotides were mostly dimers but mononucleotides were also used as required. A glass filter equipped column was charged with 20 mg of the starting nucleotide resin, Ap-d(G) (Yamasa Shoyu Co., Ltd.). After repeated washing with methylene chloride, the 4,4'-dimethoxytrityl group was eliminated by treatment with a solution of methylene chloride containing 3% trichloroacetic acid. Subsequently, the column was washed several times with 1 ml of methylene chloride. After the column was washed with anhydrous pyridine to displace the solvent, 20 mg of a nucleotide dimer, (DMTr)ApTp(NHR$_3$), (Nippon Zeon; NHR$_3$ = triethylammonium; DMTr = dimethoxytrityl) and 0.2 ml of pyridine were added, and the interior of the column was vacuum-dried with a vacuum pump. Subsequently, 20 mg of 2,4,6-trimethylbenzenesulfonyl-3-nitrotriazolide (MSNT of Wako Pure Chemical Industries, Ltd.) and 0.2 ml of anhydrous pyridine were added, and the interior of the column was displaced with a nitrogen gas. The nucleotide resin was condensed with the dimer by reaction for 45 minutes at room temperature, with occasional shaking. After completion of the reaction, the column was washed with pyridine and the unreacted OH groups were acetylated with a pyridine solution containing excess acetic anhydride and 4-dimethylaminopyridine. After washing the column with pyridine, the following dimers or monomers were condensed, in the order written, by repeating the above-described procedures: (DMTr)Ip(NHR$_3$), (DMTr)-GpGp(NHR$_3$), (DMTr)Ip(NHR$_3$), an equimolar mixture of (DMTr)CpTp(NHR$_3$) and (DMTr)TpTp(NHR$_3$), an equimolar mixture of (DMTr)ApAp(NHR$_3$) and (DMTr)ApGp(NHR$_3$), an equimolar mixture of (DMTr)ApGp-(NHR$_3$) and (DMTr)GpGp(NHR$_3$), (DMTr)GpAp(NHR$_3$), (DMTr)TpGp(NHR$_3$), an equimolar mixture of (DMTr)-ApAp(NHR$_3$) and (DMTr)GpAp(NHR$_3$), (DMTr)CpAp(NHR$_3$), an equimolar mixture of (DMTr)ApAp(NHR$_3$) and (DMTr)ApGp(NHR$_3$), (DMTr)GpCp(NHR$_3$), (DMTr)TpGp(NHR$_3$), (DMTr)Ip(NHR$_3$) and (DMTr)ApTp-(NHR$_3$), with all of these nucleotides being available from Nippon Zeon except for (DMTr)Ip(NHR$_3$) which was available from Yamasa Shoyu Co., Ltd. After completion of the reaction in the final stage, the resin was washed successively with pyridine, methylene chloride and ether without acetylation, and thereafter dried. The dried resin was suspended in 1.7 ml of a mixture of pyridine (0.5 ml), water (0.2 ml) and dioxane (1 ml) containing 1 M tetramethylguanidine and 1 M α-picolinaldoxime. The suspension was left to stand overnight at room temperature and concentrated to 100 - 200 μl under vacuum. The concentrate was mixed with a small amount (2 - 3 drops) of pyridine and 2 - 3 ml of concentrated aqueous ammonia, and the mixture was heated at 55°C for 6 hours. Following extraction with ethyl acetate, the aqueous layer was separated and concentrated under vacuum. The concentrate was dissolved in a solution of 50 mM triethyl ammonium acetate (pH 7.0) and the solution was subjected to chromatography on C-18 column (1.0 x 15 cm; Waters), with elution conducted with acetonitrile (linear density gradient of 10 - 30%) in a solution of 50 mM triethyl ammonium acetate (pH 7.0). The peak fraction eluted at an acetonitrile concentration of about 25% was concentrated under vacuum.

To the concentrate, 80% acetic acid was added and the mixture was left to stand for 30 minutes at room temperature. Following extraction with ethyl acetate, the aqueous layer was separated and concentrated under vacuum. The resulting concentrate was further purified by high performance liquid chromatography on C-18 column (from Senshu Kagaku K.K.; SSC-ODS-272; 6$^\emptyset$ mm x 200 mm). Elution was conducted with acetonitrile (10 - 20% linear density gradient) in a solution of 50 mM triethyl ammonium acetate (pH 7.0). A synthetic DNA was obtained in a yield no lower than 10A$_{260}$ units.

Analysis by the Maxam-Gilbert sequencing method [Meth. Enzym., 65, 499 (1980)] revealed that the oligonucleotide obtained had the nucleotide sequence shown in Fig. 1.

(ii) Synthesis of probe (A)

Fourteen successive nucleotides (see Fig. 1) were obtained on the basis of the sequence of 5 amino acids (Met-Pro-Ala-Phe-Ala) included within the amino acid sequence obtained in Example 3(iii).

Synthesis procedures were similar to those employed in the preparation of probe (IWQ), and the following nucleotides were condensed to a nucleotide resin, Ap-d(T) (Yamasa Shoyu Co., Ltd.) in the order written: (DMTr)CpAp(NHR$_3$), (DMTr)GpGp(NHR$_3$), an equimolar mixture of (DMTr)CpAp(NHR$_3$), (DMTr)-CpTp(NHR$_3$), (DMTr)CpGp(NHR$_3$) and (DMTr)CpCp(NHR$_3$), an equimolar mixture of (DMTr)ApGp(NHR$_3$), (DMTr)TpGp(NHR$_3$), (DMTr)GpGp(NHR$_3$) and (DMTr)CpGp(NHR$_3$), (DMTr)ApAp(NHR$_3$), an equimolar mixture of (DMTr)CpAp(NHR$_3$) and (DMTr)CpGp(NHR$_3$), and (DMTr)Gp(NHR$_3$), with all nucleotides being available from Nippon Zeon. A synthetic DNA was obtained in a yield of ca. 10A$_{260}$ units. Analysis by the Maxam-Gilbert sequencing method revealed that the oligonucleotide obtained had the nucleotide sequence shown in Fig. 1.

(iii) Synthesis of probe (LC)

Automatic DNA synthesis was accomplished with a DNA synthesizer, Model 380A of Applied Biosystems. This technique, based on the principles described by Caruthers et al. [J. Am. Chem. Soc., 103, 3185 (1981)], is generally referred to as the phosphoramidite procedure.

A phosphoramidite form of (DMTr)-dT preliminarily activated with tetrazole was condensed to dG-S (S: support) wherein 5'-dimethoxytrityl group (DMTr) was deblocked. Thereafter, the unreacted hydroxyl groups were acetylated and oxidated with iodine in the presence of water to make a phosphoryl group. After deblocking the DMTr group, condensation was repeated in the same manner until 24 nucleotides having the sequence shown in Fig. 1 were synthesized. These nucleotides were cleaved from the support, deblocked, and purified by reverse-phased high performance liquid chromatography on C-18 column (Senshu Kagaku Co., Ltd.; SSC-ODS-272).

Example 5: Cultivation of CHU-2 Cells and Preparation of mRNA

1) Cultivation and recovery of CHU-2 cells

Established CHU-2 cells were grown in a completely dense population (grown to confluency) in two culture flasks (150 cm²), recovered, suspended in 500 ml of an RPMI 1640 culture solution containing 10% of a bovine fetal serum, transferred into a glass roller bottle of 1580 cm² (Belco), and whirl-cultured for 4 days at 0.5 rpm. When the cells were found to have grown in a completely dense population (grown to confluency) on the inner wall of the roller bottle, the culture solution was removed from the roller bottle, which was charged with 100 ml of a preheated (37°C) physiological saline solution containing 0.02% of EDTA. After heating at 37°C for 2 minutes, the cells were separated from the inner wall of the flask by pipetting. The resulting cell suspension was centrifuged at 400 x g for 10 minutes to obtain a cell pellet. The cells were resuspended in 5 ml of an EDTA-free physiological saline solution. The suspension was centrifuged at 400 x g for 10 minutes to obtain a cell pellet (wet weight, ca. 0.8 g). The so obtained cells were stored frozen at -80°C until they were subjected to procedures for extraction of RNA.

2) Purification of mRNA

Isolation of mRNA from the CHU-2 cells obtained in 1) was accomplished by procedures which were essentially the same as those described in "Molecular Cloning", Maniatis et al., Cold Spring Harbor, page 196, 1982. The frozen CHU-2 cells (wet weight, 3.8 g) were suspended in 20 ml of a solution of 6 M guanidine [6 M guanidinium isothiocyanate, 5 mM sodium citrate (pH 7.0), 0.1 M $\beta$-mercaptoethanol, and 0.5% sodium sarcosyl sulfate] and the suspension was well mixed by vortexing for 2 - 3 minutes. The mixture was subjected to 10 cyclic suction and ejection with a syringe (capacity, 20 ml) equipped with a 18G needle. About 6 ml of the viscous guanidinium solution containing the disrupted cells was layered onto a 6-ml cushion of 5.7 M CsCl in 0.1 M EDTA (pH 7.5) in a Beckman SW40 Ti polyallomer centrifuge tube in such a manner that the tube became full of the contents. Four centrifuge tubes were prepared by the procedures described above and centrifuged at 120,000 x g for 15 hours at 20°C. The resulting pellets were washed three times with a small amount of 70% ethanol.

The pellets obtained from the respective tubes were combined, dissolved in 550 $\mu$l of water and worked up to provide a NaCl concentration of 0.2 M. After treatment with a 1:1 mixture of phenol and chloroform and with chloroform alone, 2.5 volumes of ethanol were added to precipitate the total RNA (ca. 10.1 mg of the total RNA was obtained from 3.8 g of wet cells).

Poly(A⁺) RNA was purified from the total RNA by the following procedures of affinity chromatography taking advantage of the attachment of a poly(A) chain at 3' terminus of the mRNA. Adsorption on oligo(dT)-cellulose (Type 7 of P-L Biochemicals) was achieved by passage through an oligo(dT)-cellulose column of the total RNA in a loading buffer [containing 10 mM Tris-HCl (pH 7.5), 0.5 M NaCl, 1 mM EDTA, and 0.1% SDS solution] after the solution had been heated at 65°C for 5 minutes. The column had been equilibrated with the same loading buffer. Elution of poly(A⁺) RNA was accomplished with a TE solution [containing 10 mM Tris-HCl (pH 7.5) and 1 mM EDTA]. The unadsorbed effluent was re-charged through the column and the eluate obtained by repeating the same procedures was mixed with the first run of eluate. As a result, 400 $\mu$g of the poly(A⁺) RNA was obtained.

The so prepared mRNA was fractionated for size by sucrose density gradient centrifugation in accordance with the procedures described in the laboratory manual of Schleif and Wensink, "Practical Methods in Molecular Biology", Springer-Verlag, New York, Heidelberg, Berlin (1981).

Stated more specifically, a 5 - 25% sucrose density gradient was created in a Beckman SW40 Ti centrifuge tube. Two sucrose solutions were prepared by dissolving 5% and 25% of RNase-free sucrose (Schwarz/Mann) in a solution containing 0.1 M NaCl, 10 mM Tris-HCl (pH 7.5), 1 mM EDTA, arid 0.5% SDS.

Eight hundred micrograms of the mRNA [poly(A$^+$)-RNA] prepared by the method already described was dissolved in 200 - 500 $\mu$l of a TE solution. The solution was heated at 65°C for 5 minutes and, after being quenched, it was placed on the sucrose density gradient solutions, which were centrifuged at 120,000 x g for 20 hours. Fractions each weighing 0.5 ml were collected and their absorption at 260 nm was measured. The sizes of the fractionated RNAs were determined on the basis of the positions of standard RNAs (ribosome RNAs 282, 18S and 5S). At the same time, the G-CSF activity of each fraction was examined with oocytes of Xenopus laevis by the following procedures. First, the mRNA of each fraction was worked up into an aqueous solution having a concentration of 1 $\mu$g/$\mu$l; oocytes were taken from Xenopus (about one year old) and the mRNA solution was injected in such a manner that a 50-ng of mRNA was injected into one oocyte; ten such oocytes were placed in each of 96 wells in a microtiter plate; the oocytes were cultured for 48 hours at room temperature in 100 $\mu$l of a Barth medium [88 mM NaCl; 1 mM KCl; 2.4 mM NaHCO$_3$; 0.82 mM MgSO$_4$; 0.33 mM Ca(NO$_3$)$_2$; 0.41 mM CaCl$_2$; 7.5 mM Tris-HCl (pH 7.6); penicillin, 10 mg/L; and streptomycin sulfate, 10 mg/L]; the supernatant of the culture was recovered, concentrated and purified to a grade suitable for assay of G-CSF activity.

The G-CSF activity was found to be present in 15 - 17S fractions.

Example 6: Synthesis of cDNA (Construction of pBR-line cDNA Library)

From the poly(A$^+$) RNA obtained in Example 5 was synthesized cDNA by the method of Land et al. [Nucleic Acids Res., 9, 2251 (1981)] as modified by the method of Gubler and Hoffman [Gene, 25, 263 (1983)].

1) Synthesis of single-stranded cDNA

An Eppendorf tube (capacity, 1.5 ml) was charged with reagents in the following order: 80 $\mu$l of a reaction buffer (500 mM KCl, 50 mM MgCl$_2$, 250 mM Tris-HCl, pH 8.3); 20 $\mu$l of 200 mM dithiothreitol, 32 $\mu$l of 12.5 mM dNTP (containing 12.5 mM each of dATP, dGTP, dCTP and dTTP), 10 $\mu$l of $\alpha$-$^{32}$PdCTP (PB 10205 of Amerscham), 32 $\mu$l of oligo(dT)$_{12-18}$ (from P-L Biochemicals; 500 $\mu$g/ml), 20 $\mu$l of poly(A$^+$) RNA (2.1 $\mu$g/$\mu$l), and 206 $\mu$l of distilled water. A total of 400 $\mu$l of the reaction solution was heated at 65°C for 5 minutes, and thereafter heated at 42°C for 5 minutes. To the heated solution, 120 units of a reverse transcriptase (Takara Shuzo Co., Ltd.) was added. Following reaction for 2 more hours at 42°C, 2 $\mu$l of an RNase inhibitor (Bethesda Research Laboratories), 20 $\mu$l of a TE solution, 16 $\mu$l of 100 mM sodium pyrophosphate and 48 units (4 $\mu$l) of a reverse transcriptase were added, and reaction was carried out at 46°C for 2 hours. The reaction was quenched by addition of 0.5 M EDTA (8 $\mu$l) and 10% SDS (8 $\mu$l). By subsequent treatment with phenol/chloroform and precipitation with ethanol (twice), a single-stranded cDNA was obtained.

2) Attachment of dC-chain to the single-stranded cDNA

The single-stranded cDNA obtained in 1) was dissolved in distilled water. To the solution was added 60 $\mu$l of a dC-chain adding buffer [400 mM potassium cacodylate, 50 mM Tris-HCl (pH 6.9), 4 mM dithiothreitol, 1 mM CoCl$_2$, and 1 mM dCTP], and the mixture was heated at 37°C for 5 minutes. To the reaction solution, 3 $\mu$l of a terminal transferase (27 units/$\mu$l; P-L Biochemicals) was added and the mixture was heated at 37°C for 2.5 minutes. Following treatment with phenol/chloroform (once) and precipitation with ethanol (twice), the dC-tailed cDNA was dissolved in 40 $\mu$l of a TE solution containing 100 mM NaCl.

3) Synthesis of double-stranded cDNA

To 40 $\mu$l of the DNA solution prepared in 2), 4 $\mu$l of oligo(dG)$_{12-18}$ (200 $\mu$g/ml; P-L Biochemicals) was added and the mixture was heated first at 65°C for 5 minutes, then at 42°C for 30 minutes. While the reaction solution was held at 0°C, 80 $\mu$l of a buffer [100 mM Tris-HCl (pH 7.5), 20 mM MgCl$_2$, 50 mM (NH$_4$)$_2$SO$_4$, and 500 mM KCl], 4 $\mu$l of 4 mM dNTP (containing 4 mM each of dATP, dCTP, dGTP and dTTP), 60 $\mu$l of 1 mM $\beta$-NAD, 210 $\mu$l of distilled water, 20 $\mu$l of E. coli DNA polymerase I (Takara Shuzo Co., Ltd.), 15 µl of E. coli DNA ligase (Takara Shuzo Co., Ltd.) and 15 µl of E. coli RNase H (Takara Shuzo Co., Ltd.) were added, and the mixture was subjected to reaction at 12°C for 1 hour. Following addition of 4 mM dNTP (4 µl), reaction was carried out at 25°C for 1 hour. By subsequent treatment with phenolchloroform and precipitation with ethanol (once), about 8 µg of a double-stranded cDNA was obtained. This double-stranded cDNA was dissolved in a TE solution and subjected to 1.2% agarose gel electrophoresis. Fragments corresponding to the size of ca. 560 bp to 2 kbp were adsorbed on Whatman DE81 and about 0.2 µg of the double-stranded cDNA could be recovered by elution.

4) Attachment of dC-chain to the double-stranded cDNA

The double-stranded cDNA prepared in 3) was dissolved in 40 µl of a TE solution. After 8 µl of a dC-tail adding buffer of the type identified in 2) had been added, the mixture was heated at 37°C for 2 minutes. Following addition of 1 µl of a terminal transferase (27 units/µl), the mixture was subjected to reaction at 37°C for 3 minutes. Thereafter, the reaction solution was immediately cooled to 0°C, and the reaction was quenched by addition of 1 µl of 0.5 M EDTA. Following treatment with phenol/chloroform and precipitation with ethanol, the precipitate obtained was suspended in 10 µl of a TE solution.

5) Construction of pBR-line cDNA library

Four microliters of a commercial oligo(dG)-tailed pBR322 vector (Bethesda Research Laboratories; 10 ng/µl) and 2 µl of the dC-tailed double-stranded cDNA obtained in 4) were annealed in a TE solution containing 75 µl of 0.1 M NaCl. The annealing consisted of three stages: heating at 65°C for 5 minutes, subsequent heating at 40°C for 2 hours, followed by cooling to room temperature.

In accordance with the method described in the laboratory manual of Maniatis et al. [Molecular Cloning, Cold Spring Harbor, p 249 ff. (1982)] (other routine techniques could also be used here), competent cells were prepared from E. coli strain X1776, and transformed with the annealed plasmid to produce transformants.

Example 7: Synthesis of cDNA (Construction of λphage Library)

1) Synthesis of single-stranded cDNA

In accordance with the procedures described in Example 5, 3.8 g of frozen CHU-2 cells were purified twice on an oligo(dT)-cellulose column and subsequently worked up to obtain 400 µg of poly(A$^+$) RNA.

A TE solution (10 µl) having 12 µg of the poly(A$^+$) RNA dissolved therein was placed in a reaction tube containing 10 µg of actinomycin D (Sigma). Thereafter, the tube was charged with reagents in the following order: 20 µl of a reverse transcription buffer [250 mM Tris-HCl (pH 8.3); 40 mM MgCl$_2$; 250 mM KCl]; 20 µl of 5 mM dNTP (containing 5 mM each of dATP, dGTP, dCTP and dTTP); 20 µl of oligo(dT)$_{12-18}$ (0.2 µg/ml; P-L Biochemicals); 1 µl of 1 M dithiothreitol; 2 µl of RNasin (30 units/µl; Promega Biotech); 10 µl of a reverse transcriptase (10 units/µl; Seikagaku Kogyo Co., Ltd.); 1 µl of α-$^{32}$P-dATP (10 µCi; Amerscham); and 16 µl of water. The reaction solution totalling a volume of 100 µl was held at 42°C for 2 hours and the reaction was quenched by addition of 0.5 M EDTA (5 µl) and 20% SDS (1 µl). By subsequent treatment with phenol/chloroform (100 µl) and precipitation with ethanol (twice), about 4 µg of a single-stranded cDNA was obtained.

2) Synthesis of double-stranded cDNA

The cDNA obtained in 1) was dissolved in 29 µl of a TE solution and a reaction solution was prepared by adding the following reagents in the order written: 25 µl of a polymerase buffer [400 mM Hepes (pH 7.6); 16 mM MgCl$_2$, 63 mM β-mercaptoethanol, and 270 mM KCl]; 10 µl of 5 mM dNTP; 1.0 µl of 15 mM β-NAD; 1.0 µl of α-$^{32}$-P-dATP (10 µCi/µl); 0.2 µl of E. coli DNA ligase (60 units/µl; Takara Shuzo Co., Ltd.); 5.0 µl of E. coli DNA polymerase I (New England Biolabs; 10 units/µl); 0.1 µl of RNase H (60 units/µl; Takara Shuzo Co., Ltd.); and 28.7 µl of distilled water.

The reaction solution was incubated at 14°C for 1 hour, allowed to return to room temperature, and incubated for an additional hour. Then, the reaction was quenched by addition of 0.5 M EDTA (5 µl) and 20% SDS (1 µl), and treatment with phenol/chloroform and precipitation with ethanol were performed. The DNA obtained was dissolved in 20 µl of 0.5 mM EDTA and a reaction solution was prepared by addition of 3 µl of a Klenow buffer [500 mM Tris-HCl (pH 8.0) and 50 mM MgCl$_2$], 3 µl of 5 mM dNTP, and 4 µl of water. After addition of 1 μl of a DNA polymerase (Klenow fragment; Takara Shuzo Co., Ltd.), the reaction solution was incubated at 30°C for 15 minutes.

The incubated reaction solution was diluted with 70 μl of a TE solution and the reaction was quenched by addition of 0.5 M EDTA (5 μl) and 20% SDS (1 μl). By subsequent treatment with phenol/chloroform and precipitation with ethanol, about 8 μg of a double-stranded cDNA was obtained.

3) Methylation of double-stranded cDNA

An aqueous solution (30 μl) of the double-stranded cDNA synthesized in 2) was mixed with 40 μl of a methylation buffer [500 mM Tris-HCl (pH 8.0); 50 mM EDTA], 20 μl of a SAM solution [800 μM S-adenosyl-L-methylmethionine (SAM); 50 mM β-mercaptoethanol], and 100 μl of water. To the mixture, 15 μl of an EcoRI methylase (New England Biolabs; 20 units/μl) was added to make a reaction solution totalling 200 μl in volume. Following incubation at 37°C for 2 hours, treatments with phenol and ether and precipitation with ethanol were conducted to recover the DNA.

4) Addition of EcoRI linker

To about 1.2 μg of the methylated double-stranded DNA, 1.5 μl of a ligase buffer [250 mM Tris-HCl (pH 7.5) and 100 mM MgCl$_2$], 0.5 μl of a preliminarily phosphorylated EcoRI linker (10mer; Takara Shuzo Co., Ltd.), 1.5 μl of 10 mM ATP, 1.5 μl of 100 mM dithiothreitol, and 2 μl of H$_2$O were added to make a reaction solution totalling 15 μl in volume. After 0.7 μl of T$_4$ DNA ligase (3.4 units/μl; Takara Shuzo Co., Ltd.) had been added, reaction was carried out overnight at 4°C. Thereafter, the ligase was inactivated by heating at 65°C for 10 minutes. The reaction solution was worked up to a total volume of 50 μl by addition of 100 mM Tris-HCl (pH 7.5), 5 mM MgCl$_2$, 50 mM NaCl and 100 μg/ml of gelatin. Following addition of EcoRI (3.5 μl; 10 units/μl), reaction was carried out at 37°C for 2 hours. Subsequently, 2.5 μl of 0.5 M EDTA and 0.5 μl of 20% SDS were added, followed by treatment with phenol/chloroform and precipitation with ethanol so as to recover the DNA. Thereafter, the unreacted EcoRI linker was removed by gel filtration on Ultrogel AcA34 (LKB) or agarose-gel electrophoresis, so as to recover about 0.5 - 0.7 μg of the linker-added double-stranded cDNA.

5) Joining double-stranded cDNA to λgt10 vector

The linker-added double-stranded cDNA was mixed with 2.4 μg of preliminarily EcoRI-treated λgt10 vector (Vector Cloning system), 1.4 μl of a ligase buffer (250 mM Tris-HCl and 100 mM MgCl$_2$), and 6.5 μl of distilled water, and the mixture was heated at 42°C for 15 minutes. Thereafter, 1 μl of 10 mM ATP, 1 μl of 0.1 M dithiothreitol and 0.5 μl of T$_4$ DNA ligase were added to make a total volume of 15 μl and reaction was carried out overnight at 12°C.

6) In vitro packaging

About a third of the recombinant DNAs prepared in 5) was packed with an in vitro packaging kit (Promega Biotech) to obtain phage plaques.

Example 8: Screening of pBR-Line Library with Probe (IWQ)

Whatman 541 paper was placed on a colony-growing agar medium and left to stand at 37°C for 2 hours. The filter paper was subsequently treated by the following method of Taub and Thompson [Anal. Biochem., 126, 222 (1982)].

The colonies transferred onto the 541 paper were further grown onto an agar medium containing chloramphenicol (250 μg/μl) overnight at 37°C.

The 541 paper was recovered and left at room temperature for 3 minutes on another sheet of filter paper that had been impregnated with a 0.5 N NaOH solution. This procedure was repeated twice. Two similar runs were conducted for 3 minutes using a solution of 0.5 M Tris-HCl (pH 8). At 4°C, treatments were conducted with a solution of 0.05 M Tris-HCl (pH 8) for 3 minutes, and with 1.5 mg/ml of a lysozyme solution [containing 0.05 M Tris-HCl (pH 8) and 25% sucrose] for 10 minutes; then, at 37°C, treatments were conducted with a solution of 1 x SSC (0.15 M NaCl and 0.015 M sodium citrate) for 2 minutes, and with a 1 x SSC solution containing 200 μg/ml of proteinase K for 30 minutes; finally, at room temperature, treatments were conducted with a 1 x SSC solution for 2 minutes, and with 95% ethanol solution for 2 minutes. The final step was repeated twice. Thereafter, the 541 paper was dried. The dried 541 paper was immersed in a 25:24:1 mixture of phenol/chloroform/isoamylalcohol [equilibrated with 100 mM Tris-HCl (pH 8.5), 100 mM NaCl and 10 mM EDTA] for 30 minutes at room temperature. Subsequently, similar procedures were repeated three times with a 5 x SSC solution for 3 minutes, then twice with a 95% ethanol solution for 3 minutes. Thereafter, the filter paper was dried.

The probe (IWQ) was labelled with $^{32}$P in accordance with routine procedures (see Molecular Cloning) and colony hybridization was performed in accordance with the method of Wallace et al. [Nucleic Acids Res., 9, 879 (1981)]. Prehybridization was conducted at 65°C for 4 hours in a hybridization buffer containing 6 x NET [0.9 M NaCl; 0.09 M Tris-HCl (pH 7.5); and 6 mM EDTA], 5 x Denhardt's solution, 0.1% SDS and 0.1 mg/ml of denatured DNA (calf thymus). Thereafter, hybridization was conducted overnight at 56°C in a hybridization buffer (for its formulation, see above) containing 1 x 10$^6$ cpm/ml of the radiolabelled probe (IWQ). After completion of the reaction, the 541 paper was washed twice with a 6 x SSC solution (containing 0.1% SDS) for 30 minutes at room temperature, then washed at 56°C for 1.5 minutes. The washed 541 paper was then subjected to autoradiography.

The plasmid was separated from positive clones and subjected to Southern blotting with the probe (IWQ). Hybridization and autoradiography were conducted under the same conditions as described above.

Similarly, Southern blotting was conducted with the probe (A). Using a hybridization buffer having the formulation shown above, hybridisation was conducted first at 49°C for 1 hour. After leaving it to 39°C, hybridisation was further continued at the same temperature for 1 hour. After completion of the reaction, a nitrocellulose filter was washed twice with 6 x SSC containing 0.1% SDS for 30 minutes at room temperature, then washed at 39°C for 3 minutes. The washed paper was subjected to autoradiography.

As a result, a single clone was found to be positive. Nucleotide sequencing by the dideoxy method revealed that this clone had a DNA composed of 308 base pairs containing the portions of both probe (IWQ) and probe (A) (Fig. 2). The pBR322-derived plasmid containing this insert was named pHCS-1.

Example 9: Screening of λPhage Line Library with pHCS-1 Derived DNA Probe

Plaque hybridization was conducted in accordance with the method of Benton and Davis [Science, 196, 180 (1977)]. The pHCS-1 obtained in Example 8 was treated with Sau3A and EcoRI to obtain a DNA fragment of ca. 600 bp. This DNA fragment was radiolabelled by nick translation in accordance with routine procedures. A nitrocellulose filter (S & S) was placed on the phage plaque-growing agar medium to transfer the phages onto the filter. After denaturing the phage DNA with 0.5 M NaOH, the filter paper was treated by the following procedures: treatment with 0.1 M NaOH and 1.5 M NaCl for 20 seconds; two treatments with 0.5 M Tris-HCl (pH 7.5) and 1.5 M NaCl for 20 seconds; finally, treatment with 120 mM NaCl, 15 mM sodium citrate, 13 mM KH$_2$PO$_4$ and 1 mM EDTA (pH 7.2) for 20 seconds.

The filter was subsequently dried and heated at 80°C for 2 hours to immobilize the DNA. Prehybridization was conducted overnight at 42°C in a prehybridization buffer containing 5 x SSC, 5 x Denhardt's solution, 50 mM phosphate buffer, 50% formamide, 0.25 mg/ml of denatured DNA (salmon sperm DNA) and 0.1% SDS. Thereafter, hybridization was conducted at 42°C for 20 hours in a hybridization buffer containing 4 x 10$^5$ cpm/ml of pHCS-1 probe that had been radiolabelled by nick translation. This hybridization buffer was a mixture of 5 x SSC, 5 x Denhardt's solution, 20 mM phosphate buffer (pH 6.0), 50% formamide, 0.1% SDS, 10% dextran sulfate and 0.1 mg/ml of denatured DNA (salmon sperm DNA).

The hybridized nitrocellulose filter was washed for 20 minutes with 2 x SSC containing 0.1% SDS at room temperature, then for 30 minutes with 0.1 x SSC containing 0.1% SDS at 44°C, and finally for 10 minutes with 0.1 x SSC at room temperature. Detection by autoradiography was then conducted.

As a result, five positive clones (G1 - G5) were obtained. The clone containing a "full-length" cDNA was checked for its DNA nucleotide sequence by the dideoxy method and the nucleotide sequence shown in Fig. 3 was identified. This cDNA was cut out of the λgt10 vector and joined to pBR327 [Soberon et al., Gene, 9, 287 (1980)] at the EcoRI site to form a plasmid which could be prepared on a large scale. This plasmid is named pBRG4.

Example 10: Screening of λPhage Line Library with pBRG4-Derived DNA Probe and Probe (LC)

Plaque hybridization was performed in accordance with the method of Benton and Davis (see Science, ibid.) employed in Example 9. A nitrocellulose filter (S & S) was placed on the phage plaque-growing agar medium to transfer the phages onto the filter. After denaturing the phage DNA with 0.5 M NaOH, the filter was treated by the following procedures: treatment with 0.1 M NaOH and 1.5 M NaCl for 20 seconds; then two treatments with 0.5 M Tris-HCl (pH 7.5) and 1.5 M NaCl for 20 seconds; finally, treatment with 120 mM NaCl, 15 mM sodium citrate, 13 mM $KH_2PO_4$ and 1 mM EDTA (pH 7.2) for 20 seconds. The filter was subsequently dried, and heated at 80°C for 2 hours to immobilize the DNA. Two sheets of the same filter were prepared in the manner described above and subjected to screening with the pBRG4-derived DNA probe and the probe (LC).

Screening with the pBRG4-derived DNA probe was carried out by the following procedures. The pBRG4 was treated with EcoRI to obtain a DNA fragment of ca. 1500 bp. This DNA fragment was radiolabelled by nick translation in accordance with routine procedures. One of the two nitrocellulose filters was subjected to prehybridization overnight at 42°C in a prehybridization buffer containing 5 x SSC, 5 x Denhardt's solution, 50 mM phosphate buffer, 50% formamide, 0.25 mg/ml of denatured DNA (salmon sperm DNA) and 0.1% SDS. Thereafter, the filter was subjected to hybridization at 42°C for 20 hours in a hybridization buffer containing the radiolabelled DNA probe (ca. $1 \times 10^6$ cpm/ml) of ca. 1500 bp. This hybridization buffer was a mixture of 5 x SSC, 5 x Denhardt's solution, 20 mM phosphate buffer (pH 6.0), 50% formamide, 0.1% SDS, 10% dextran sulfate and 0.1 mg/ml of denatured DNA (salmon sperm DNA). The hybridized nitrocellulose filter was washed for 20 minutes with 2 x SSC containing 0.1% SDS at room temperature, then for 30 minutes with 0.1 x SSC containing 0.1% SDS at 44°C, and finally for 10 minutes with 0.1 x SSC at room temperature. Detection by autoradiography was then conducted.

Screening with the probe (LC) was carried out by the following procedures. The other filter was preliminarily treated with 3 x SSC containing 0.1% SDS at 65°C for 2 hours. Then, prehybridization was conducted at 65°C for 2 hours in a solution containing 6 x NET, 1 x Denhardt's solution, and 100 $\mu$g/ml of denatured DNA (salmon sperm DNA). Hybridization was subsequently conducted overnight at 63°C in a hybridization buffer containing the radiolabelled probe (LC) ($2 \times 10^6$ cpm/ml). This hybridization buffer was also a mixture of 6 x NET, 1 x Denhardt's solution and 100 $\mu$g/ml of denatured DNA (salmon sperm DNA). The hybridized nitrocellulose filter was washed three times (20 minutes each) with 6 x SSC containing 0.1% SDS at room temperature, then washed with 6 x SSC containing 0.1% SDS at 63°C for 2 minutes.

The filter was dried and detection was conducted by autoradiography.

In the screening described above, clones which were positive to both probes were selected and the clone containing a "full-length" cDNA was checked for its nucleotide sequence by the dideoxy method. It was found to have the nucleotide sequence shown in Fig. 4. This cDNA was cut out of the λgt10 vector and joined to pBR327 at the EcoRI site to prepare a plasmid pBRV2.

Example 11: Screening of Human Chromosomal Gene Library

1) Construction of human chromosomal gene library

The human chromosomal gene library which was provided by courtesy of Dr. Maniatis of Harvard University had been prepared by the following procedures: the whole chromosomal DNA was extracted from the human fetal liver with phenol or other appropriate chemicals and partially digested with restriction enzymes, HaeIII and AluI; the resulting DNA fragments were treated by sucrose density gradient centrifugation to concentrate the fragments having chain lengths of about 18 - 25 kb; the concentrated fragments were joined to the arm DNA of E. coli phage λ Charon 4A, with short-chained synthetic nucleotides having the cleavage sites of the restriction enzyme EcoRI being inserted, so as to prepare infectious phage DNA recombinants; with a view to providing enhanced infectiousness, more refined phage λ particles were created by packaging. The so prepared human gene library is theoretically considered to be a set of recombinants containing human DNAs with chain lengths of 18 - 25 kb which contained practically all human genes.

2) Screening of human chromosomal gene library with the pHCS-1 derived DNA probe

Plaque hybridization was conducted in accordance with the method of Benton and Davis [Science, 196, 180 (1977)]. The pHCS-1 obtained in Example 8 was treated with Sau3A and EcoRI to obtain a DNA fragment of ca. 600 bp. This DNA fragment was radiolabelled by nick translation in accordance with routine procedures. A nitrocellulose filter (S & S) was placed on the phage plaque-growing agar medium to transfer the phages onto the filter. After denaturing the phage DNA with 0.5 M NaOH, the filter paper was treated by the following procedures: treatment with 0.1 M NaOH and 1.5 M NaCl for 20 seconds; two treatments with 0.5 M Tris-HCl (pH 7.5) and 1.5 M NaCl for 20 seconds; finally, treatment with 120 mM NaCl, 15 mM sodium citrate, 13 mM $KH_2PO_4$ and 1 mM EDTA (pH 7.2) for 20 seconds.

The filter was subsequently dried and heated at 80°C for 2 hours to immobilize the DNA. Prehybridization was conducted overnight at 42°C in a prehybridization buffer containing 5 x SSC, 5 x Denhardt's solution, 50 mM phosphate buffer, 50% formamide, 0.25 mg/ml of denatured DNA (salmon sperm DNA) and 0.1% SDS. Thereafter, hybridization was conducted at 42°C for 20 hours in a hybridization buffer containing 4 x 10⁵ cpm/ml of pHCS-1 probe that had been radiolabelled by nick translation. This hybridization buffer was a mixture of 5 x SSC, 5 x Denhardt's solution, 20 mM phosphate buffer (pH 6.0), 50% formamide, 0.1% SDS, 10% dextran sulfate and 0.1 mg/ml of denatured DNA (salmon sperm DNA).

The hybridized nitrocellulose filter was washed for 20 minutes with 2 x SSC containing 0.1% SDS at room temperature, then for 30 minutes with 0.1 x SSC containing 0.1% SDS at 44°C, and finally for 10 minutes with 0.1 x SSC at room temperature. Detection by autoradiography was then conducted.

As a result, ten-odd positive clones were obtained. Recombinant DNAs were prepared from these clones by the method of Maniatis [Cell, 15, 687 (1978)]. The obtained DNAs were treated with restriction enzymes such as EcoRI, BamHI and BglII, analyzed by agarose gel electrophoresis, and their restriction enzyme map was prepared in accordance with the method of Fritsch et al. (see Cell, ibid.)

Southern hybridization was conducted with the probe being the radiolabelled pHCS-1 derived DNA fragment that was the same as what was used in the above-described screening procedures. A DNA fragment of ca. 8 kb that was cut with EcoRI was selected from the clones that hybridized with the probe. This fragment was subcloned to the EcoRI site of pBR327. The subcloned DNA was subjected to another treatment with restriction enzymes and Southern hybridization was conducted repeatedly. A DNA fragment of ca. 4 kb that was cut out with EcoRI and XhoI was found to contain a gene coding for the human G-CSF polypeptide. This DNA fragment was checked for the sequence of its ca. 3-kb portion by the dideoxy method and the nucleotide sequence shown in Fig. 5 was identified. This DNA fragment had the restriction enzyme cleavage sites shown in Fig. 6.

Screening of human chromosomal genes was also conducted using pBRG4-derived DNA and pBRV2-derived DNA as probes. In either case, a DNA fragment of 1500 bp that had been treated with EcoRI was directly radiolabelled by nick translation in the manner described above or, alternatively, a DNA fragment of ca. 700 bp that was obtained by successive treatments with EcoRI and DraI was radiolabelled by nick translation. The so prepared probe was used in plaque hybridization that was conducted under the same conditions as described above. Selected clones were analyzed by Southern hybridization so as to obtain a DNA fragment having the nucleotide sequence shown in Fig. 5. The plasmid thus obtained was named pBRCE3β.

Example 12: Construction of Recombinant Vector Containing Chromosomal Gene for Expression in COS Cells

The plasmid pBRCE3β that was obtained in Example 11 and which contained the chromosomal gene shown in Fig. 5 was treated with EcoRI. The pSVH⁺K⁺ plasmid described by Banerji et al. in Cell, 27, 299 (1981) was treated with KpnI to remove the globin gene. The plasmid was further subjected to partial digestion with HindIII so as to remove part of the late gene of SV40. The fragments were rejoined to prepare an expression vector pML-E⁺.

This vector was treated with the restriction enzyme, EcoRI, and dephosphorylated with an alkaline phosphatase (Takara Shuzo Co., Ltd.) to obtain a vector DNA, which was linked to the aforementioned chromosomal DNA with the aid of a T4DNA ligase (Takara Shuzo Co., Ltd.) to obtain pMLCE3α. As shown in Fig. 7, this plasmid contained the enhancer of SV40 gene, the replication origin of SV40, the replication origin of pBR322 and the pBR322-derived β-lactamase gene (Ampʳ), and had the human G-CSF chromosomal gene joined downstream from the enhancer of SV40 gene.

Example 13: Expression of Human G-CSF Chromosomal Gene in COS Cells

COS-1 cells (provided by courtesy of Dr. Gluzman of Cold Spring Harbor Laboratory, U.S.A.) that had been grown to a density of about 70% in Petri dishes (9 cm⁰, Nunc) using a DMEM medium (Dulbecco's modified Eagle's medium available from Nissui Seiyaku K.K. under the trade name "Nissui") containing 10% calf serum were transformed by either the calcium phosphate procedure [Wigler et al., Cell, 14, 725 (1978)] or the DEAE-dextran:chloroquine method [see, for example, Gordon et al., Science, 228, 810 (1985)-].

Transformation by the calcium phosphate procedure was conducted as follows: 160 μg of the plasmid pMLCE3α prepared in Example 12 was dissolved in 320 μl of a TE solution and, after addition of distilled water (3.2 ml), 504 μl of 2 M CaCl₂ was added.

To the resulting solution, 4 ml of 2 x HBS (50 mM Hepes, 280 mM NaCl, 1.5 mM phosphate buffer, pH 7.12) was added and the mixture was cooled on ice for 20 - 30 minutes. The cooled mixture was added dropwise to the medium in an amount of 1 ml per Petri dish where the COS-1 cells had grown. After cultivation for 4 hours at 37°C in a CO₂ incubator, the cells were washed with a serum-free DMEM medium, then left to stand for about 3 minutes at room temperature in 5 ml of a DMEM medium containing 20% glycerol, and rewashed with a serum-free DMEM medium. After the serum-free DMEM medium was removed, 10 ml of a DMEM medium containing 10% calf serum was added and cultivation was conducted overnight in a CO₂ incubator. After the medium was replaced by a fresh one of the same type, cultivation was conducted for an additional 3 days.

Transformation by the DEAE-dextran:chloroquine method was conducted as follows: as in the calcium phosphate procedure, COS-1 cells were cultivated to grow to a density of 70% and washed twice with a serum-free DMEM medium; to the washed cells, a serum-free DMEM medium containing 250 $\mu$g/ml of DEAE-dextran and 2 $\mu$g/ml of the plasmid pMLCE3$\alpha$ prepared in Example 12 was added and cultivation was conducted at 37°C for 12 hours; subsequently, the cells were washed twice with a serum-free DMEM medium and subjected to further cultivation at 37°C for 2 hours in a DMEM medium containing 10% calf serum and 1 mM chloroquine; thereafter, the cells were washed twice with a serum-free DMEM medium and cultured at 37°C for an additional 3 days in a DMEM medium containing 10% calf serum.

The supernatant of the so obtained culture of COS-1 cells was adjusted to a pH of 4 with 1 N acetic acid. After addition of an equal volume of n-propanol, the resulting precipitate was removed by centrifugation. The supernatant was passed through an open column (1$^{\emptyset}$ cm x 2 cm$^{L}$) filled with a C8 reverse-phased carrier (Yamamura Kagaku K.K.) and elution was conducted with 50% n-propanol. The eluate was diluted two-fold with water and subjected to reverse-phased high performance liquid chromatography on YMC-C8 column (Yamamura Kagaku K.K.), followed by elution with n-propanol (30 - 60% linear density gradient) containing 0.1% TFA. The fractions which were eluted at n-propanol concentrations of about 40% were recovered, freeze-dried and dissolved in 0.1 M glycidine buffer (pH 9). As a result of these procedures, the human G-CSF in the supernatant of the culture of COS-1 cells was concentrated about 20-fold.

As controls, COS-1 cells were transformed with G-CSF chromosomal-gene free pML-E$^{+}$ by the above-described procedures and the supernatant of the resulting culture was concentrated.

The human G-CSF activities of the obtained samples were assayed by the "Method of Human G-CSF Activity Assay (a)" described earlier in this specification. The results are summarized in Table 1.

## Table 1

|  | Human neutrophilic colonies (colonies/dish) |
|---|---|
| Purified human G-CSF (20 ng) | 18 |
| Culture of COS cells transformed with pML-E$^{+}$ (concentrated 20-fold) | 0 |
| Culture of COS cells transformed with pMLCE3$\alpha$ (concentrated 20-fold) | 23 |
| Culture of COS cells transformed with pMLCE3$\alpha$ (concentrated 10-fold) | 19 |

Example 14: RNA Analysis of G-CSF

COS cells cultivated to a cell concentration of 8 x 10⁶ cells/plate (9 cm$^{\emptyset}$) were transformed with 80 $\mu$g of the plasmid pMLCE3$\alpha$. After 48 hours, the total RNA was prepared in accordance with the procedure of Chirgwin [Biochemistry, 18, 5294 - 5299 (1979)].

The plasmid pBRG4 obtained in Example 9 was cleaved with restriction enzyme AhaIII and the resulting pBRG4-derived DNA fragment was radiolabelled with [$\gamma$-³²P]ATP using T4 polynucleotide kinase to obtain an ca. 2.8-kb DNA fragment containing G-CSF cDNA. The fragment was recovered and used as a DNA probe. After the DNA probe ($1.5 \times 10^5$ c.p.m., $2.8 \times 10^6$ c.p.m./$\mu$g DNA) was denatured, it was mixed with 20 $\mu$g of the total RNA prepared from COS cells. Hybridization at 45°C for 15 hours was conducted. The mixture was digested with 200 units/ml or 400 units/ml of SI nuclease (P.L. Biochemicals) in accordance with the procedures of Weaver and Weissmann [Nucleic Acid Res., 7, 1175 - 1193 (1979)], followed by 4% polyacrylamide gel electrophoresis in the presence of 8.3 M urea. Detection by autoradiography was then conducted.

As a result, a band corresponding to 722 bp was observed as a strongly radiolabelled band in COS cells, from which a band corresponding to 487 bp was also detected.

Therefore, the RNA of COS cells was found to contain G-CSF mRNAs that corresponded to the amino acid sequences depicted in Fig. 3 and Fig. 4, respectively.

Example 15: Physicochemical Properties of the Obtained G-CSF

1) Analysis of amino acid composition

The crude CSF sample prepared in Example 13 was purified in accordance with the procedures described in Example 2(iii). The purified CSF sample was hydrolyzed by routine procedures, and the amino acid composition of the protein portion of the hydrolyzate was analyzed by a method of amino acid analysis with an automatic amino acid analyzer, Hitachi 835 (Hitachi Ltd.) The results are shown in Table 2. Hydrolysis was conducted under the following conditions:

(i) 6 N HCl, 110°C, 24 hours, in vacuum
(ii) 4 N methanesulfonic acid + 0.2% 3-(2-aminoethyl)indole, 110°C, 24 hours, 48 hours, 72 hours, in vacuum

The sample was dissolved in a solution (1.5 ml) containing 40% n-propanol and 0.1% trifluoroacetic acid. Aliquots each weighing 0.1 ml were dried with a dry nitrogen gas and, after addition of the reagents listed in (i) or (ii), the containers were sealed in vacuum, followed by hydrolysis of the contents.

Each of the values shown in Table 2 was the average of four measurements, 24 hour value for (i) and 24, 48 and 72 hour values for (ii), except that the contents of Thr, Ser, 1/2 Cys, Met, Val, Ile and Trp were calculated by the following methods (see "Tampaku Kagaku (Protein Chemistry) II", A Course in Biochemical Experiments, Tokyo Kagaku Dohjin):

-- For Thr, Ser, 1/2 Cys and Met, the time-dependent profile of the 24, 48 and 72 hour values for (ii) was extrapolated by zero hours.
-- For Val and Ile, the 72 hour value for (ii) was used.
-- For Trp, the average of 24, 48 and 72 hour values for (ii) was used.

## Table 2

### Amino Acid Analysis Data

| Amino acids | Mole% |
|---|---|
| Asp (Asp + Asn) | 2.3 |
| Thr | 3.9 |
| Ser | 8.3 |
| Glu (Glu + Gln) | 15.3 |
| Pro | 7.4 |
| Gly | 7.9 |
| Ala | 10.8 |
| 1/2 Cys | 2.8 |
| Val | 4.3 |
| Met | 1.7 |
| Ile | 2.3 |
| Leu | 18.7 |
| Tyr | 1.7 |
| Phe | 3.4 |
| Lys | 2.3 |
| His | 2.9 |
| Trp | 1.1 |
| Arg | 2.9 |

2) Sugar composition analysis

An internal standard (25 nmol of inositol) was added to 200 ng of the purified CSF sample used in the analysis of amino acid composition 1). After addition of a methanol solution (500 $\mu$l) containing 1.5 N HCl, reaction was carried out at 90°C for 4 hours in a $N_2$ purged, closed tube. After the tube was opened, silver carbonate ($Ag_2CO_3$) was added to neutralize the contents. Thereafter, 50 $\mu$l of acetic anhydride was added and the tube was shaken for an adequate period. Subsequently, the tube was left overnight in the dark at room temperature. The upper layer was put into a sample tube and dried with a nitrogen gas. Methanol was added to the precipitate and the mixture was washed and lightly centrifuged. The upper layer was put into the same sample tube and dried. After addition of 50 $\mu$l of a TMS reagent (5:1:1 mixture of pyridine, hexamethyl disilazane and trimethylchlorosilane), reaction was carried out at 40°C for 20 minutes and the reaction product was stored in a deep freezer. A standard was prepared by combining 25 nmol of inositol with 50 nmol each of galactose (Gal), N-acetyl galactosamine (Gal NAc), sialic acid and any other appropriate reagents.

The samples thus prepared were subjected to gas chromatographic analysis under the following conditions:

Conditions of analysis

| | |
|---|---|
| Column : | 2% OV - 17 VINport HP, 60 - 80 mesh, 3 m, glass |
| Temperature : | elevated from 110 to 250°C at 4°C/min. |
| Carrier gas ($N_2$) pressure : | initially 1.2 - 1.6 kg/cm$^2$ |
| | finally 2 - 2.5 kg/cm$^2$ |
| Sensitivity : | 10 MΩ range, 0.1 - 0.4 volts |
| Pressure : | $H_2$, 0.8 kg/cm$^2$ |
| | air, 0.8 kg/cm$^2$ |
| Sample feed : | 2.5 - 3.0 µl. |

As a result of the analysis, galactose, N-acetyl galactosamine and sialic acid were identified in the CSF sample of the present invention.

3) Molecular weight

The molecular weight of the CSF sample used in the analysis of amino acid composition 1) was determined by sodium dodecylsulfate-polyacrylamide gel electrophoresis (SDS-PAGE). The electrophoretic equipment was PROTEAN$^{TH}$ (16 cm, product of Bio-Rad Corporation), using a gel made up of a polyacrylamide slab gel (T = 15%, C = 2.6%) measuring 140 mm x 160 mm x 1.5 mm, and a concentrating gel (T = 3%, C = 20%). A denatured CSF sample was prepared by the following procedure: CSF was boiled for 3 minutes in a solution containing 2% of sodium dodecylsulfate in 0.46 M 2-mercaptoethanol. After performing electrophoresis with 4 µg of the sample with a constant current of 30 mA for 4 hours, the gel was removed and stained with 0.25% Coomassie Brilliant Blue R 250 (product of Sigma Chemical Co.) for band detection. The following substances were used as molecular weight markers after similar treatments: phosphorylase B (mol. wt. 92,500), bovine serum albumin (BSA, mol. wt. 67,000), ovalbumin (OVA, mol. wt. 45,000), carbonic anhydrase (mol. wt. 31,000), soybean trypsin inhibitor (mol. wt. 21,500) and lysozyme (mol wt. 14,400).

As a result, a single band correponding to a molecular weight of 19,000 ± 1,000 was detected from the CSF sample.

4) Isoelectric point

The purified CSF sample used in the analysis of amino acid composition in (1) was employed.

The isoelectric point of the CSF of the present invention was determined by a flat bed, isoelectric electrophoretic apparatus, FBE-3000 (product of Pharmacia Fine Chemicals). After 2-hour electrophoresis with a constant power of 30 watts (Vmax = 2,000 volts) on a polyacrylamide gel (T = 5%, C = 3%, 115 mm x 230 mm) containing Pharmalyte (pH = 4 - 6.5, Pharmacia Fine Chemicals) and 4M urea, the CSF was fixed with 30% methanol/10% trichloroacetic acid/35% sulfosalicylic acid, and stained with Coomassie Brilliant Blue R-250. A Low pI kit (pH: 2.5 - 6.5, product of Pharmacia Fine Chemicals) was used as a isoelectric point marker.

Analysis of band separation at a pH of 4 to 6.5 gave three distinct bands corresponding to pI = 5.5, 5.8 and 6.1 for the CSF sample.

Example 16: Construction of Recombinant Vector Containing Chromosomal Gene for Expression in C127 Cells

The plasmid pMLCE3α obtained in Example 12 was treated with EcoRI and a fragment of ca. 4 kb was recovered by the procedures described in Molecular Cloning, ibid. The recovered fragment was used as a source of the chromosomal G-CSF gene.

The fragment was treated with the Klenow fragment of DNA polymerase I to create blunt ends (A).

In a separate way, the EcoRI fragment prepared in Example 9 which had the cDNA shown in Fig. 3 was treated with a restriction enzyme, DraI, at 37°C for 2 hours, followed by treatment with the Klenow fragment of DNA polymerase I (Takara Shuzo Co., Ltd.) to create blunt ends. One microgram of BglII linker (8mer, Takara Shuzo Co., Ltd.) was phosphorylated with ATP and joined to about 1 µg of the separately obtained mixture of DNA fragments. The joined fragments were treated with a restriction enzyme, BglII, and subjected to agarose gel electrophoresis. Subsequently, only the largest DNA fragment was recovered.

This DNA fragment was equivalent to about 710 base pairs containing a human G-CSF polypeptide coding portion. A vector pdKCR [Fukunaga et al., Proc. Natl. Acad. Sci., USA, 81, 5086 (1984)] was treated with a restriction enzyme, BamHI, and subsequently dephosphorylated with an alkali phosphatase (Takara Shuzo Co., Ltd.). The vector DNA obtained was joined to the 710-bp cDNA fragment in the presence of $T_4$ DNA ligase (Takara Shuzo Co., Ltd.), so as to produce pHGA410.

The promoter of SV40 (ca. 0.4-kb EcoRI-EcoRI fragment) was cut out from this plasmid pHGA410 by the procedures described in Molecular Cloning, ibid., and was subsequently treated with the Klenow fragment of DNA polymerase (B).

In a separate step, a plasmid pdBPV-1 having a bovine papilloma virus (BPV) [this plasmid was obtained by courtesy of Dr. Howley and is described in Sarver, N., Sbyrne, J.C. & Howley, P.M., Proc. Natl. Acad. Sci., USA, 79, 7147-7151 (1982)] was treated with HindIII and PvuII to obtain a DNA fragment of ca. 8.4 kb. This fragment was treated with the Klenow fragment of DNA polymerase I and dephosphorylated with a bacterial alkaline phosphatase (C).

The DNA fragments (A), (B) and (C) each weighing 0.1 $\mu$g were dissolved in 20 $\mu$l of a reaction solution [50 mM Tris-HCl (pH 7.6), 10 mM $MgCl_2$, 10 mM DTT, 1 mM ATP] and reaction was carried out overnight at 4°C in the presence of 180 units of a T4DNA ligase.

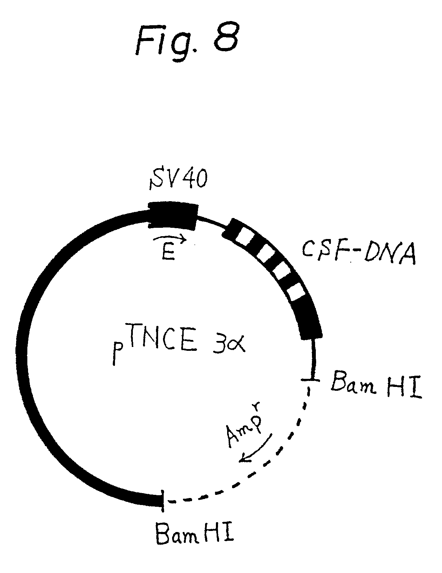

The reaction solution was subsequently treated by the rubidium chloride procedure described in Molecular Cloning, ibid. so as to obtain the plasmid pTNCE3$\alpha$ (Fig. 8).

The resulting pTNCE3$\alpha$ was an expression vector that had a chromosomal CSF gene linked downstream from the early promoter of SV40 and which contained a 65% portion of BPV.

The DNA fragment (A) used as a source of the chromosomal G-CSF gene may be replaced by a DNA fragment of ca. 1.78 kb that is obtained by the following procedures: 20 $\mu$g of pMLCE3$\alpha$ is dissolved in 100 $\mu$l of a mixture of 10 mM Tris-HCl (pH 8.0), 7 mM $MgCl_2$, 100 mM NaCl, 7 mM 2-mercaptoethanol and 0.01% BSA; the solution is incubated at 37°C for 5 hours in the presence of 20 units of StuI and subjected to electrophoresis through 1.2% agarose gel.

Example 17: Transformation of C127 Cells and G-CSF Expression Therein

Before it was used to transform mouse C127 cells, the pTNCE3$\alpha$ obtained in Example 16 was treated with a restriction enzyme, BamHI. Twenty micrograms of the plasmid pTNCE3$\alpha$ was dissolved in 100 $\mu$l of a reaction solution [10 mM Tris-HCl (pH 8.0), 7 mM $MgCl_2$, 100 mM NaCl, 2 mM 2-mercaptoethanol and 0.01% BSA] and treated with 20 units of BamHI (Takara Shuzo Co., Ltd.), followed by treatments with phenol and ether, and precipitation with ethanol.

Mouse C127I cells were grown in a Dulbecco's minimal essential medium containing 10% bovine fetal serum (Gibco). The C127I cells growing on plates (5 cm$^\emptyset$) were transformed with 10 $\mu$g, per plate, of the separately prepared DNA by the calcium phosphate procedure [see Haynes, J. & Weissmann, C., Nucleic Acids Res., 11, 687-706 (1983)]. After treatment with glycerol, the cells were incubated at 37°C for 12 hours.

The incubated cells were transferred onto three fresh plates (5 cm$^\emptyset$) and the media were changed twice a week. At day 16, the foci were transferred onto fresh plates and subjected to serial cultivation on a Dulbecco's minimal essential medium containing 10% bovine fetal serum (Gibco), so as to select clones having high G-CSF production rate.

Example 18: Construction of Recombinant Vector Containing Chromosomal Gene for Expression in CHO Cells

(1) Construction of the plasmid pD26SVCE3$\alpha$

As in the case of expression in C127 cells, the plasmid pMLCE3$\alpha$ was treated with StuI and a DNA fragment of ca. 1.78 kb was recovered; alternatively, the same plasmid was treated with EcoRI and an EcoRI fragment of about 4 kb was recovered. Either fragment was suitable for use as a source of the chromosomal G-CSF gene.

The source fragment was treated with the Klenow fragment of DNA polymerase I (a).

As in Example 16, the promoter of SV40 (EcoRI-EcoRI fragment) was cut out from pHGA410 to obtain a fragment of about 0.4 kb, which was similarly treated with the Klenow fragment of DNA polymerase I (b).

In a separate step, the plasmid pAdD26SVpA [Kaufman, R.G. & Sharp, P.A., Mol. Cell. Biol., 2, 1304-1319 (1982)] was treated with EcoRI, then with the Klenow fragment of DNA polymerase, and finally dephosphorylated by treatment with a bacterial alkaline phosphatase (c).

The fragments, (a), (b) and (c), each weighing 0.1 μg were dissolved in 20 μl of a reaction solution [50 mM Tris-HC1 (pH 7.6), 10 mM MgCl₂, 10 mM DTT, 1 mM ATP] and reaction was carried out overnight at 4°C in the presence of 180 units of a T4DNA ligase.

The reaction solution was subsequently treated by the rubidium chloride precedure described in Molecular Cloning, ibid., so as to transform E. coli strain DHI. The resulting Tet$^r$ colonies were screened for those containing the plasmid pD26SVCE3α.

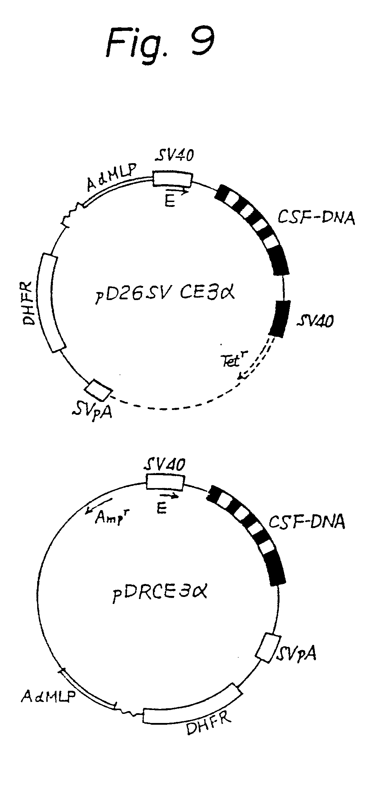

As shown in Fig. 9, the plasmid pD26SVCE3α has the CSF gene linked to the early gene of SV40, and the dhfr gene linked downstream from the principal late promoter of adenovirus.

(2) Construction of the plasmid pDRCE3α

The plasmid pAdD26SVpA was treated with EcoRI and BamHI so as to obtain a DNA fragment (ca. 2 kb) containing dhfr gene (d).

In a separate step, the plasmid pHGA410 (Example 16) was partially digested with EcoRI to create blunt ends. A linker HindIII was attached to the DNA, which was subsequently treated with HindIII and T4DNA ligase. The treated DNA was used to transform E. coli strain DHI by the rubidium chloride procedure. The resulting plasmid was named pHGA410(H). From the pHGA410(H), EcoRI - SalI fragment was prepared (e).

The fragments (d), (a) obtained in (I) and (e) were linked so as to construct an Amp$^r$ expression vector pDRCE3α (Fig. 9).

Example 19: Transformation of CHO Cells and G-CSF Expression Therein

CHO cells were transformed with the so obtained plasmids, pD26SVCE3α and pDRCE3α by the following procedures.

CHO cells (dhfr⁻ strain; obtained by courtesy of Dr. L. Chasin of Columbia University) were cultivated for growth in alpha-minimal essential medium containing 10% calf serum (α-MEN supplemented with adenosine, deoxyadenosine and thymidine) in plates (9 cm$^∅$, Nunc). The cultured cells were transformed by the calcium phosphate procedure [Wigler et al., Cell, 14, 725 (1978)] in the following manner.

A carrier DNA (calf thymus DNA) was added in an appropriate amount to 1 μg of the plasmid pD26SVCE3α prepared in 1) of Example 18, and the mixture was dissolved in 375 μl of a TE solution, followed by addition of 125 μl of 1 M CaCl₂. After the solution was cooled on ice for 3 - 5 minutes, 500 μl of 2 x HBS (50 mM Hepes, 280 mM NaCl, and 1.5 mM phosphate buffer) was added to the solution. After re-cooling on ice, the solution was mixed with 1 ml of the culture of CHO cells, transferred onto plates, and incubated for 9 hours in a CO₂ incubator. The medium was removed from the plate and, following washing with TBS (Tris-buffered saline), addition of 20% glycerol-containing TBS, and re-washing, a non-selective medium (the α-MEN medium described above except that it was supplemented with nucleotides) was added. After 2-day incubation, a 10-fold dilution of the culture was transferred onto a selective medium (not supplemented with nucleotides). The cultivation was continued, with the medium being replaced by a fresh selective medium every 2 days, and the resulting colonies were selected and transferred onto fresh plates, where the cells grew in the presence of 0.02 μM methotrexate (MTX), followed by cloning through growth in the presence of 0.05 μM MTX, which was later increased to 0.1 μM.

The transformation of CHO cells was also accomplished with the plasmid pDRCE3α by increasing MTX concentrations so as to obtain G-CSF producing colonies.

Example 20: Assay of the G-CSF Activity of Transformants (expressing human chromosomal gene)

The supernatants of cultures of C127 cells and CHO cells which were obtained in Examples 17 and 19, respectively, were worked up as in Example 13 to obtain human G-CSF and its activity was assayed. The results are shown in Table 3.

## Table 3

### Assay of Human G-CSF Activity

| | | Human neutrophilic colonies (colonies/dish) |
|---|---|---|
| Purified human G-CSF (20 ng) | | 85 |
| BPV | Culture of C127 cells transformed with pdBPV-1 (concentrated 20-fold) | 0 |
| | Culture of C127 cells transformed with pTNCE3α (concentrated 20-fold) | 83 |
| dhfr | Culture of CHO cells transformed with pAdD26SVpA (concentrated 20-fold) | 0 |
| | Culture of CHO cells transformed with pD26SVCE3α (concentrated 20-fold) | 85 |
| | Culture of CHO cells transformed with pDRCE3α (concentrated 20-fold) | 86 |

Cell line CHU-2 was deposited with Institut Pasteur on September 12, 1985 and received the deposition No. I-483

## Claims

1. A human chromosomal gene coding for a polypeptide having a human granulocyte colony stimulating factor activity having all or part of the nucleotide sequence shown in Figure 5.

2. A human chromosomal gene according to Claim 1 wherein said human chromosomal gene contains a nucleotide sequence that takes part in transcriptional control.

3. A human chromosomal gene according to Claim 1 which is connected to a microorganism- or virus-derived replicon.

4. A recombinant vector containing a human chromosomal gene according to claim 1.

5. A recombinant vector according to Claim 4 wherein said human chromosomal gene contains a nucleotide sequence that takes part in transcriptional control.

6. A recombinant vector according to Claim 4 or 5 wherein said human chromosomal gene is connected to a microorganism-or virus-derived replicon.

7. A transformant containing a recombinant vector according to any one of claims 4 to 6.

8. A process for producing a glycoprotein having a human granulocyte colony stimulating factor activity which comprises:
    a) culturing the transformant according to claim 7 in a culture medium and
    b) recovering from the culture a glycoprotein having the human granulocyte colony stimulating factor activity.

9. A process according to Claim 8 wherein said glycoprotein has a sugar chain portion and a polypeptide which is represented by all or part of the following amino acid sequence:

```
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  (Val Ser  Glu)ₘCys  Ala  Thr  Tyr  Lys  Leu
Cys  His  Pro  Glu  Glu  Leu  Val  Leu  Leu  Gly  His
Ser  Leu  Gly  Ile  Pro  Trp  Ala  Pro  Leu  Ser  Ser
Cys  Pro  Ser  Gln  Ala  Leu  Gln  Leu  Ala  Gly  Cys
Leu  Ser  Gln  Leu  His  Ser  Gly  Leu  Phe  Leu  Tyr
Gln  Gly  Leu  Leu  Gln  Ala  Leu  Glu  Gly  Ile  Ser
Pro  Glu  Leu  Gly  Pro  Thr  Leu  Asp  Thr  Leu  Gln
Leu  Asp  Val  Ala  Asp  Phe  Ala  Thr  Thr  Ile  Trp
Gln  Gln  Met  Glu  Glu  Leu  Gly  Met  Ala  Pro  Ala
Leu  Gln  Pro  Thr  Gln  Gly  Ala  Met  Pro  Ala  Phe
Ala  Ser  Ala  Phe  Gln  Arg  Arg  Ala  Gly  Gly  Val
Leu  Val  Ala  Ser  His  Leu  Gln  Ser  Phe  Leu  Glu
Val  Ser  Tyr  Arg  Val  Leu  Arg  His  Leu  Ala  Gln
Pro , and
```

where m is 0 or 1 .

10. E. coli strain X 1776 harboring a ca. 4 kb DNA fragment coding for human G-CSF inserted into the EcoRI site of plasmid pBR327 (FERM BP-956).

## Revendications

1. Gène chromosomique humain codant pour un polypeptide ayant une activité de facteur de stimulation de colonie de granulocytes humains comprenant tout ou partie de la séquence de nucléotides que montre la Fig. 5.

2. Gène chromosomique humain suivant la revendication 1, dans lequel le gène chromosomique humain contient une séquence de nucléotides qui prend part au contrôle transcriptionnel.

3. Gène chromosomique humain suivant la revendication 1, qui est uni à un réplicon dérivant d'un microorganisme ou d'un virus.

4. Vecteur recombinant contenant un gène chromosomique humain suivant la revendication 1.

5. Vecteur recombinant suivant la revendication 4, dans lequel le gène chromosomique humain contient une séquence de nucléotides qui prend part au contrôle transcriptionnel.

6. Vecteur recombinant suivant la revendication 4 ou 5, dans lequel le gène chromosomique humain est uni à un réplicon dérivant d'un micro-organisme ou d'un virus.

7. Transformant contenant un vecteur recombinant suivant l'une quelconque des revendications 4 à 6.

8. Procédé de production d'une glycoprotéine ayant une activité de facteur de stimulation de colonie de granulocytes humains, qui comprend :
   a) la culture du transformant suivant la revendication 7 dans un milieu de culture, et
   b) l'isolement, à partir de la culture, d'une glycoprotéine ayant l'activité de facteur de stimulation de colonie de granulocytes humains.

9. Procédé suivant la revendication 8, dans lequel la glycoprotéine comprend une partie de chaîne de sucre et une partie polypeptide qui est représentée par tout ou partie de la séquence d'acides aminés ci-après :

```
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  (Val Ser Glu)ₘ Cys  Ala  Thr  Tyr  Lys  Leu
Cys  His  Pro  Glu  Glu  Leu  Val  Leu  Leu  Gly  His
Ser  Leu  Gly  Ile  Pro  Trp  Ala  Pro  Leu  Ser  Ser
Cys  Pro  Ser  Gln  Ala  Leu  Gln  Leu  Ala  Gly  Cys
Leu  Ser  Gln  Leu  His  Ser  Gly  Leu  Phe  Leu  Tyr
Gln  Gly  Leu  Leu  Gln  Ala  Leu  Glu  Gly  Ile  Ser
Pro  Glu  Leu  Gly  Pro  Thr  Leu  Asp  Thr  Leu  Gln
Leu  Asp  Val  Ala  Asp  Phe  Ala  Thr  Thr  Ile  Trp
Gln  Gln  Met  Glu  Glu  Leu  Gly  Met  Ala  Pro  Ala
Leu  Gln  Pro  Thr  Gln  Gly  Ala  Met  Pro  Ala  Phe
Ala  Ser  Ala  Phe  Gln  Arg  Arg  Ala  Gly  Gly  Val
Leu  Val  Ala  Ser  His  Leu  Gln  Ser  Phe  Leu  Glu
Val  Ser  Tyr  Arg  Val  Leu  Arg  His  Leu  Ala  Gln
Pro, et
```

où m est 0 ou 1.

10. Souche X 1776 d'E. coli hébergeant un fragment d'ADN d'environ 4 kilobases codant pour le G-SCF humain inséré dans le site EcoRI du plasmide pBR327 (FERM BP-956).

## Patentansprüche

1. Menschliches chromosomales Gen, das ein Polypeptid mit Aktivität von menschliche Granulozyten-Kolonie-stimulierender Faktor codiert, das die gesamte oder einen Teil der in Figur 5 dargestellten Nucleotidsequenz aufweist.

27

2. Menschliches chromosomales Gen nach Anspruch 1, wobei das menschliche chromosomale Gen eine Nucleotidsequenz enthält, die an der transkriptionalen Kontrolle beteiligt ist.

3. Menschliches chromosomales Gen nach Anspruch 1, das mit einem von einem Mikroorganismus oder Virus abgeleiteten Replikon verbunden ist.

4. Rekombinanter Vektor, der ein menschliches chromosomales Gen nach Anspruch 1 enthält.

5. Rekombinanter Vektor nach Anspruch 4, wobei das menschliche chromosomale Gen eine Nucleotidsequenz enthält, die an der transkriptionalen Kontrolle beteiligt ist.

6. Rekombinanter Vektor nach Anspruch 4 oder 5, wobei das menschliche chromosomale Gen mit einem von einem Mikroorganismus oder Virus abgeleiteten Replikon verbunden ist.

7. Transformante, die einen rekombinanten Vektor nach einem der Ansprüche 4 bis 6 enthält.

8. Verfahren zur Herstellung eines Glykoproteins mit der Aktivität von menschliche Granulocyten-Kolonie-stimulierendem Faktor, umfassend
   a) Züchtung der Transformante nach Anspruch 7 in einem Kulturmedium und
   b) Gewinnung eines Glykoproteins mit der Aktivität von menschliche Granulocyten-Kolonie-stimulierendem Faktor aus der Kultur.

9. Verfahren nach Anspruch 8, wobei das Glykoprotein einen Zuckerketten-Anteil und ein Polypeptid enthält, das durch die gesamte oder einen Teil der folgenden Aminosäuresequenz dargestellt wird:

| | | | | | | | | | | |
|---|---|---|---|---|---|---|---|---|---|---|
| Thr | Pro | Leu | Gly | Pro | Ala | Ser | Ser | Leu | Pro | Gln |
| Ser | Phe | Leu | Leu | Lys | Cys | Leu | Glu | Gln | Val | Arg |
| Lys | Ile | Gln | Gly | Asp | Gly | Ala | Ala | Leu | Gln | Glu |
| Lys | Leu | (Val | Ser | Glu)$_m$ | Cys | Ala | Thr | Tyr | Lys | Leu |
| Cys | His | Pro | Glu | Glu | Leu | Val | Leu | Leu | Gly | His |
| Ser | Leu | Gly | Ile | Pro | Trp | Ala | Pro | Leu | Ser | Ser |
| Cys | Pro | Ser | Gln | Ala | Leu | Gln | Leu | Ala | Gly | Cys |
| Leu | Ser | Gln | Leu | His | Ser | Gly | Leu | Phe | Leu | Tyr |
| Gln | Gly | Leu | Leu | Gln | Ala | Leu | Glu | Gly | Ile | Ser |
| Pro | Glu | Leu | Gly | Pro | Thr | Leu | Asp | Thr | Leu | Gln |
| Leu | Asp | Val | Ala | Asp | Phe | Ala | Thr | Thr | Ile | Trp |
| Gln | Gln | Met | Glu | Glu | Leu | Gly | Met | Ala | Pro | Ala |
| Leu | Gln | Pro | Thr | Gln | Gly | Ala | Met | Pro | Ala | Phe |
| Ala | Ser | Ala | Phe | Gln | Arg | Arg | Ala | Gly | Gly | Val |
| Leu | Val | Ala | Ser | His | Leu | Gln | Ser | Phe | Leu | Glu |
| Val | Ser | Tyr | Arg | Val | Leu | Arg | His | Leu | Ala | Gln |
| Pro | , | | | | | | | | | |

wobei m 0 oder 1 ist.

10. E. coli Stamm X 1776, der ein menschlichen G-CSF codierendes, etwa 4 kb großes DNA-Fragment enthält, das an der EcoRI-Stelle von Plasmid pBR327 eingefügt ist (FERM BP-956).

# Fig. 1

## Probe (IWQ)

Ile Trp Gln Gln Met Glu Glu Leu Gly Met

```
5'---- ATI TGG CAA CAA ATG GAA GAA CTI GG1 ATG ----3'
            G   G           G   G T
```

## Probe (A)

Met Pro Ala Phe Ala

```
5'---- ATG CCA GCA TTT GC  ----3'
           T   T   C
           G   G
           C   C
```

```
3'---- TAC GGA CGA AAA CG  ----5'
           T   T   G                ] Probe(A)
           G   G
           C   C
```

## Probe (LC)

Gln Glu Lys Leu Cys Ala Thr Tyr

```
5'---- CAG GAG AAG CTG TGT GCC ACC TAC ----3'

3'---- GTC CTC TTC GAC ACA CGG TGG ATG ----5'  Probe(LC)
```

# Fig. 2

```
   CC  CTG  GAA  GGG  ATC  TCC  CCC  GAG

TTG  GGT  CCC  ACC  TTG  GAC  ACA  CTG

CAG  CTG  GAC  GTC  GCC  GAC  TTT  GCC

ACC  ACC  ATC  TGG  CAG  CAG  ATG  GAA

GAA  CTG  GGA  ATG  GCC  CCT  GCC  CTG

CAG  CCC  ACC  CAG  GGT  GCC  ATG  CCG

GCC  TTC  GCC  TCT  GCT  TTC  CAG  CGC

CGG  GCA  GGA  GGG  GTC  CTA  GTT  GCC

TCC  CAT  CTG  CAG  AGC  TTC  CTG  GAG

GTG  TCG  TAC  CGC  GTT  CTA  CGC  CAC

CTT  GCC  CAG  CCC  TGA  GCC  AAG  CCC

TCC  CCA  TCC  CAT  GTA  TTT  ATC  TCT

ATT  TAA  TAT  TTA  TGT  CTA  TTT
```

# Fig. 3-1

```
                                                              10                              30
                                           CGGAGCCTGCAGCCCAGCCCCACCCAGACCC
```

```
                     50                                70  GCC CTG CAG CTG CTG CTG TGG            90
ATG GCT GGA CCT GCC ACC CAG AGC CCC ATG AAG CTG ATG GCC CTG CAG CTG CTG CTG TGG
Met Ala Gly Pro Ala Thr Gln Ser Pro Met Lys Leu Met Ala Leu Gln Leu Leu Leu Trp
-30                                              -20
```

```
                     110                              130                            150
CAC AGT GCA CTC TGG ACA GTG CAG GAA GCC ACC CCC CTG GGC CCT GCC AGC TCC CTG CCC
His Ser Ala Leu Trp Thr Val Gln Glu Ala Thr Pro Leu Gly Pro Ala Ser Ser Leu Pro
-10                                              -1   1                               10
```

```
                     170                              190                            210
CAG AGC TTC CTG CTC AAG TGC TTA GAG CAA GTG AGG AAG ATC CAG GGC GAT GGC GCA GCG
Gln Ser Phe Leu Leu Lys Cys Leu Glu Gln Val Arg Lys Ile Gln Gly Asp Gly Ala Ala
                                       20                                            30
```

```
                     230                              250                            270
CTC CAG GAG AAG CTG GTG AGT GAG TGT GCC ACC TAC AAG CTG TGC CAC CCC GAG GAG CTG
Leu Gln Glu Lys Leu Val Ser Glu Cys Ala Thr Tyr Lys Leu Cys His Pro Glu Glu Leu
                                       40                                            50
```

```
                     290                              310                            330
GTG CTG CTC GGA CAC TCT CTG GGC ATC CCC TGG GCT CCC CTG AGC AGC TGC CCC AGC CAG
Val Leu Leu Gly His Ser Leu Gly Ile Pro Trp Ala Pro Leu Ser Ser Cys Pro Ser Gln
                                       60                                            70
```

```
                     350                              370                            390
GCC CTG CAG CTG GCA GGC TGC TTG AGC CAA CTC CAT AGC GGC CTT TTC CTC TAC CAG GGG
Ala Leu Gln Leu Ala Gly Cys Leu Ser Gln Leu His Ser Gly Leu Phe Leu Tyr Gln Gly
                                       80                                            90
```

EP 0 220 520 B1

# Fig. 3-2

```
                  410                              430                              450
CTC CTG CAG GCC CTG GAA GGG ATC TCC CCC GAG TTG GGT CCC ACC TTG GAC ACA CTG CAG
Leu Leu Gln Ala Leu Glu Gly Ile Ser Pro Glu Leu Gly Pro Thr Leu Asp Thr Leu Gln
                                       100                                      110


                  470                              490                              510
CTG GAC GTC GCC GAC TTT GCC ACC ACC ATC TGG CAG CAG ATG GAA GAA CTG GGA ATG GCC
Leu Asp Val Ala Asp Phe Ala Thr Thr Ile Trp Gln Gln Met Glu Glu Leu Gly Met Ala
                                       120                                      130


                  530                              550                              570
CCT GCC CTG CAG CCC ACC CAG GGT GCC ATG CCG GCC TTC GCC TCT GCT TTC CAG CGC CGG
Pro Ala Leu Gln Pro Thr Gln Gly Ala Met Pro Ala Phe Ala Ser Ala Phe Gln Arg Arg
                                       140                                      150


                  590                              610                              630
GCA GGA GGG GTC CTG GTT GCC TCC CAT CTG CAG AGC TTC CTG GAG GTG TCG TAC CGC GTT
Ala Gly Gly Val Leu Val Ala Ser His Leu Gln Ser Phe Leu Glu Val Ser Tyr Arg Val
                                       160                                      170


                  650                      670                      690
CTA CGC CAC CTT GCC CAG CCC TGA GCCAAGCCCTCCCCATCCCATGTATTTATCTCTATTTAATATTTATG
Leu Arg His Leu Ala Gln Pro End


     710                 730                 750                 770
TCTATTTAAGCCTCATATTTAAAGACAGGGAAGAGCAGAACGGAGCCCCAGGCCTCTGTGTCCTTCCCTGCATTTCTG


     790                 810                 830                 850
AGTTTCATTCTCCTGCCTGTAGCAGTGAGAAAAAGCTCCTGTCCTCCCATCCCCTGGACTGGGAGGTAGATAGGTAAAT


     870                 890                 910                 930
ACCAAGTATTTATTACTATGACTGCTCCCCAGCCCTGGCTCTGCAATGGGCACTGGGATGAGCCGCTGTGAGCCCCTGG
```

# Fig. 3-3

TCCTGAGGGTCCCCACCTGGGACCCTTGAGAGTATCAGGTCTCCCACGTGGGAGACAAGAAATCCCTGTTTAATATTTA

AACAGCAGTGTTCCCCATCTGGGTCCTTGCACCCCTCACTCTGGCCTCAGCCGACTGCACAGCGGCCCCTGCATCCCCTT

GGCTGTGAGGCCCCTGGACAAGCAGAGGTGGCCAGAGCTGGGAGGCATGGCCCTGGGGTCCCACGAATTTGCTGGGGAA

TCTCGTTTTTCTTCTTAAGACTTTTGGGACATGGTTTGACTCCCGAACATCACCGACGCGTCTCCTGTTTTTCTGGGTG

GCCTCGGGACACCTGCCCTGCCCCCACGAGGGTCAGGACTGTGACTCTTTTTAGGGCCAGGCAGGTGCCTGGACATTTG

CCTTGCTGGACGGGGACTGGGGATGTGGGAGGGAGCAGACAGGAGGAATCATGTCAGGCCTGTGTGTGAAAGGAAGCTC

CACTGTCACCCTCCACCTCTTCACCCCCCACTCACCAGTGTCCCCTCCACTGTCACATTGTAACTGAACTTCAGGATA

ATAAAGTGTTTGCCTCCAAAAAAAAAAAAAAAAAAAAAAAAA

EP 0 220 520 B1

EP 0 220 520 B1

*Fig. 4-1*

```
                                                      10                        30
                                        GGAGCCTGCAGCCCAGCCCCACCCAGACCC

                        50                              70                        90
ATG GCT GGA CCT GCC ACC CAG AGC CCC ATG AAG CTG ATG GCC CTG CAG CTG CTG CTG TGG
Met Ala Gly Pro Ala Thr Gln Ser Pro Met Lys Leu Met Ala Leu Gln Leu Leu Leu Trp
-30                                               -20

                        110                             130                       150
CAC AGT GCA CTC TGG ACA GTG CAG GAA GCC ACC CCC CTG GGC CCT GCC AGC TCC CTG CCC
His Ser Ala Leu Trp Thr Val Gln Glu Ala Thr Pro Leu Gly Pro Ala Ser Ser Leu Pro
-10                                           -1   1                               10

                        170                             190                       210
CAG AGC TTC CTG CTC AAG TGC TTA GAG CAA GTG AGG AAG ATC CAG GGC GAT GGC GCA GCG
Gln Ser Phe Leu Leu Lys Cys Leu Glu Gln Val Arg Lys Ile Gln Gly Asp Gly Ala Ala
                                           20                                     30

                        230                             250                       270
CTC CAG GAG AAG CTG TGT GCC ACC TAC AAG CTG TGC CAC CCC GAG GAG CTG GTG CTG CTC
Leu Gln Glu Lys Leu Cys Ala Thr Tyr Lys Leu Cys His Pro Glu Glu Leu Val Leu Leu
                                           40                                     50

                        290                             310                       330
GGA CAC TCT CTG GGC ATC CCC TGG GCT CCC CTG AGC AGC TGC CCC AGC CAG GCC CTG CAG
Gly His Ser Leu Gly Ile Pro Trp Ala Pro Leu Ser Ser Cys Pro Ser Gln Ala Leu Gln
                                           60                                     70

                        350                             370                       390
CTG GCA GGC TGC TTG AGC CAA CTC CAT AGC GGC CTT TTC CTC TAC CAG GGG CTC CTG CAG
Leu Ala Gly Cys Leu Ser Gln Leu His Ser Gly Leu Phe Leu Tyr Gln Gly Leu Leu Gln
                                           80                                     90
```

# Fig. 4-2

```
                    410                                    430                                 450
GCC CTG GAA GGG ATC TCC CCC GAG TTG GGT CCC ACC TTG GAC ACA CTG CAG CTG GAC GTC
Ala Leu Glu Gly Ile Ser Pro Glu Leu Gly Pro Thr Leu Asp Thr Leu Gln Leu Asp Val
                                        100                                               110


                    470                                    490                                 510
GCC GAC TTT GCC ACC ACC ATC TGG CAG CAG ATG GAA GAA CTG GGA ATG GCC CCT GCC CTG
Ala Asp Phe Ala Thr Thr Ile Trp Gln Gln Met Glu Glu Leu Gly Met Ala Pro Ala Leu
                                        120                                               130


                    530                                    550                                 570
CAG CCC ACC CAG GGT GCC ATG CCG GCC TTC GCC TCT GCT TTC CAG CGC CGG GCA GGA GGG
Gln Pro Thr Gln Gly Ala Met Pro Ala Phe Ala Ser Ala Phe Gln Arg Arg Ala Gly Gly
                                        140                                               150


                    590                                    610                                 630
GTC CTA GTT GCC TCC CAT CTG CAG AGC TTC CTG GAG GTG TCG TAC CGC GTT CTA CGC CAC
Val Leu Val Ala Ser His Leu Gln Ser Phe Leu Glu Val Ser Tyr Arg Val Leu Arg His
                                        160                                               170


                    650                      670                      690
CTT GCC CAG CCC TGA GCCAAGCCCTCCCCATCCCATGTATTTATCTCTATTTAATATTTATGTCTATTTAAGCC
Leu Ala Gln Pro End


    710                      730                      750                      770
TCATATTTAAAGACAGGGAAGAGCAGAACGGAGCCCCAGGCCTCTGTGTCCTTCCCTGCATTTCTGAGTTTCATTCTCC


    790                      810                      830                      850
TGCCTGTAGCAGTGAGAAAAAGCTCCTGTCCTCCCATCCCCTGGACTGGGAGGTAGATAGGTAAATACCAAGTATTTAT
```

EP 0 220 520 B1

# Fig. 4-3

TACTATGACTGCTCCCCAGCCCTGGCTCTGCAATGGGCACTGGGATGAGCCGCTGTGAGCCCCTGGTCCTGAGGGTCCC

CACCTGGGACCCTTGAGAGTATCAGGTCTCCCACGTGGGAGACAAGAAATCCCTGTTTAATATTTAAACAGCAGTGTTC

CCCATCTGGGTCCTTGCACCCCTCACTCTGGCCTCAGCCGACTGCACAGCGGCCCCTGCATCCCCTTGGCTGTGAGGCC

CCTGGACAAGCAGAGGTGGCCAGAGCTGGGAGGCATGGCCCTGGGGTCCCACGAATTTGCTGGGGAATCTCGTTTTTCT

TCTTAAGACTTTTGGGACATGGTTTGACTCCCGAACATCACCGACGCGTCTCCTGTTTTTCTGGGTGGCCTCGGGACA

CCTGCCCTGCCCCCACGAGGGTCAGGACTGTGACTCTTTTTAGGGCCAGGCAGGTGCCTGGACATTTGCCTTGCTGGAC

GGGGACTGGGGATGTGGGAGGGAGCAGACAGGAGGAATCATGTCAGGCCTGTGTGTGAAAGGAAGCTCCACTGTCACCC

TCCACCTCTTCACCCCCCACTCACCAGTGTCCCCTCCACTGTCACATTGTAACTGAACTTCAGGATAATAAAGTGCTTG

CCTCCAAAAAAAAAAAAAAAAAAAAAAAAAA

EP 0 220 520 B1

# Fig. 5 - 1

CTGCCGCTTCCAGGCGTCTATCAGCGGCTCAGCCTTTGTTCAGCTGTTCTGTTCAAACACTCTGGGGCCATT

CAGGCCTGGGTGGGGCAGCGGGAGGAAGGGAGTTTGAGGGGGGCAAGGCGACGTCAAAGGAGGATCAGAGATTCC

ACAATTTCACAAAACTTTCGCAAACAGCTTTTTGTTCCAACCCCCCTGCATTGTCTTGGACACCAAATTTGCATA

AATCCTGGGAAGTTATTACTAAGCCTTAGTCGTGGCCCCAGGTAATTTCCTCCCAGGCCTCCATGGGGTTATGTA

TAAAGGGCCCCCTAGAGCTGGGCCCCAAAACAGCCCGGAGCCTGCAGCCCAGCCCCACCCAGACCC ATG GCT
                                                                    Met Ala
                                                                    -30

GGA CCT GCC ACC CAG AGC CCC ATG AAG CTG ATG G GTGAGTGTCTTGGCCCAGGATGGGAGAG
Gly Pro Ala Thr Gln Ser Pro Met Lys Leu Met A
                                        -20

CCGCCTGCCCTGGCATGGGAGGGAGGCTGGTGTGACAGAGGGGCTGGGGATCCCCGTTCTGGGAATGGGGATTAA

AGGCACCCAGTGTCCCCGAGAGGGCCTCAGGTGGTAGGGAACAGCATGTCTCCTGAGCCCGCTCTGTCCCCAG

CC CTG CAG CTG CTG CTG TGG CAC AGT GCA CTC TGG ACA GTG CAG GAA GCC ACC CCC
la Leu Gln Leu Leu Leu Trp His Ser Ala Leu Trp Thr Val Gln Glu Ala Thr Pro
               -10                                              -1   1

# Fig. 5-2

EP 0 220 520 B1

```
                 650                                    670                                    690
CTG GGC CCT GCC AGC TCC CTG CCC CAG AGC TTC CTG CTC AAG TGC TTA GAG CAA GTG
Leu Gly Pro Ala Ser Ser Leu Pro Gln Ser Phe Leu Leu Lys Cys Leu Glu Gln Val
                              10                                                       20

                 710                                    730                                    750
AGG AAG ATC CAG GGC GAT GGC GCA GCG CTC CAG GAG AAG CTG GTG AGT GAG GTGGGTG
Arg Lys Ile Gln Gly Asp Gly Ala Ala Leu Gln Glu Lys Leu(Val Ser Glu)
                                        30

                 770                         790                         810
AGAGGGCTGTGGAGGGAAGCCCGGTGGGGAGAGCTAAGGGGGATGGAACTGCAGGGCCAACATCCTCTGGAAGGG

      830                   850                      870                   890
ACATGGGAGAATATTAGGAGCAGTGGAGCTGGGGAAGGCTGGGAAGGGACTTGGGGAGGAGGACCTTGGTGGGGA

      910                   930                      950                   970
CAGTGCTCGGGAGGGCTGGCTGGGATGGGAGTGGAGGCATCACATTCAGGAGAAAGGGCAAGGGCCCCTGTGAGA

         990                   1010                   1030                   1050
TCAGAGAGTGGGGGTGCAGGGCAGAGAGGAACTGAACAGCCTGGCAGGACATGGAGGGAGGGGAAAGACCAGAGA

           1070                   1090                   1110
GTCGGGGAGGACCCGGGAAGGAGCGGCGACCCGGCCACGGCGAGTCTCACTCAGCATCCTTCCATCCCCAG TGT
                                                                           Cys

      1130                        1150                            1170
GCC ACC TAC AAG CTG TGC CAC CCC GAG GAG CTG GTG CTG CTC GGA CAC TCT CTG GGC
Ala Thr Tyr Lys Leu Cys His Pro Glu Glu Leu Val Leu Leu Gly His Ser Leu Gly
 40                                                   50
```

## Fig. 5-3

```
          1190                                1210                              1230
ATC CCC TGG GCT CCC CTG AGC AGC TGC CCC AGC CAG GCC CTG CAG CTG GTGAGTGTCA
Ile Pro Trp Ala Pro Leu Ser Ser Cys Pro Ser Gln Ala Leu Gln Leu
   60                                                 70
```

```
          1250              1270              1290              1310
GGAAAGGATAAGGCTAATGAGGAGGGGGAAGGAGAGGAGGAACACCCATGGGCTCCCCCATGTCTCCAGGTTCCA
```

```
            1330              1350              1370
AGCTGGGGGCCTGACGTATCTCAGGCAGCACCCCCTAACTCTTCCGCTCTGTCTCACAG GCA GGC TGC TTG
                                                              Ala Gly Cys Leu
```

```
   1390                            1410                          1430
AGC CAA CTC CAT AGC GGC CTT TTC CTC TAC CAG GGG CTC CTG CAG GCC CTG GAA GGG
Ser Gln Leu His Ser Gly Leu Phe Leu Tyr Gln Gly Leu Leu Gln Ala Leu Glu Gly
   80                                            90
```

```
      1450                        1470                          1490
ATC TCC CCC GAG TTG GGT CCC ACC TTG GAC ACA CTG CAG CTG GAC GTC GCC GAC TTT
Ile Ser Pro Glu Leu Gly Pro Thr Leu Asp Thr Leu Gln Leu Asp Val Ala Asp Phe
      100                                          110
```

```
         1510                    1530                  1550
GCC ACC ACC ATC TGG CAG CAG GTGAGCCTTGTTGGGCAGGGTGGCCAAGGTCGTGCTGGCATTCTGGG
Ala Thr Thr Ile Trp Gln Gln
            120
```

```
1570              1590              1610              1630
CACCACAGCCGGGCCTGTGTATGGGCCCTCTCCATGCTGTCAGCCCCCAGCATTTCCTCATTTGTAATAACGCCC
```

EP 0 220 520 B1

# Fig. 5-4

ACTCAGAAGGGCCCAACCACTGATCACAGCTTTCCCCCACAG ATG GAA GAA CTG GGA ATG GCC CCT
                                           Met Glu Glu Leu Gly Met Ala Pro
                                                                        130

GCC CTG CAG CCC ACC CAG GGT GCC ATG CCG GCC TTC GCC TCT GCT TTC CAG CGC CGG
Ala Leu Gln Pro Thr Gln Gly Ala Met Pro Ala Phe Ala Ser Ala Phe Gln Arg Arg
                                    140                                   150

GCA GGA GGG GTC CTG GTT GCC TCC CAT CTG CAG AGC TTC CTG GAG GTG TCG TAC CGC
Ala Gly Gly Val Leu Val Ala Ser His Leu Gln Ser Phe Leu Glu Val Ser Tyr Arg
                                        160

GTT CTA CGC CAC CTT GCC CAG CCC TGA GCCAAGCCCTCCCCATCCCATGTATTTATCTCTATTTAA
Val Leu Arg His Leu Ala Gln Pro End
170

TATTTATGTCTATTTAAGCCTCATATTTAAAGACAGGGAAGAGCAGAACGGAGCCCCAGGCCTCTGTGTCCTTCC

CTGCATTTCTGAGTTTCATTCTCCTGCCTGTAGCAGTGAGAAAAAGCTCCTGTCCTCCCATCCCCTGGACTGGGA

GGTAGATAGGTAAATACCAAGTATTTATTACTATGACTGCTCCCCAGCCCTGGCTCTGCAATGGGCACTGGGATG

AGCCGCTGTGAGCCCCTGGTCCTGAGGGTCCCCACCTGGGACCCTTGAGAGTATCAGGTCTCCCACGTGGGAGAC

EP 0 220 520 B1

# Fig. 5-5

```
      2190                2210                2230                2250
     AAGAAATCCCTGTTTAATATTTAAACAGCAGTGTTCCCCATCTGGGTCCTTGCACCCCTCACTCTGGCCTCAGCC

       2270                2290                2310                2330
     GACTGCACAGCGGCCCCTGCATCCCCTTGGCTGTGAGGCCCCTGGACAAGCAGAGGTGGCCAGAGCTGGGAGGCA

          2350                2370                2390                2410
     TGGCCCTGGGGTCCCACGAATTTGCTGGGGAATCTCGTTTTTCTTCTTAAGACTTTTGGGACATGGTTTGACTCC

                2430                2450                2470
     CGAACATCACCGACGTGTCTCCTGTTTTTCTGGGTGGCCTCGGGACACCTGCCCTGCCCCCACGAGGGTCAGGAC

     2490          2510                2530                2550
       TGTGACTCTTTTTAGGGCCAGGCAGGTGCCTGGACATTTGCCTTGCTGGATGGGGACTGGGGATGTGGGAGGGAG

        2570                2590                2610                2630
     CAGACAGGAGGAATCATGTCAGGCCTGTGTGTGAAAGGAAGCTCCACTGTCACCCTCCACCTCTTCACCCCCCAC

          2650                2670                2690                2710
     TCACCAGTGTCCCCTCCACTGTCACATTGTAACTGAACTTCAGGATAATAAAGTGTTTGCCTCCAGTCACGTCCT

                2730                2750                2770
     TCCTCCTTCTTGAGTCCAGCTGGTGCCTGGCCAGGGGCTGGGGAGGTGGCTGAAGGGTGGGAGAGGCCAGAGGGA

     2790          2810                2830                2850
       GGTCGGGGAGGAGGTCTGGGGAGGAGGTCCAGGGAGGAGGAGGAAAGTTCTCAAGTTCGTCTGACATTCATTCCG

        2870                2890                2910                2930
     TTAGCACATATTTATCTGAGCACCTACTCTGTGCAGACGCTGGGCTAAGTGCTGGGGACACAGCAGGGAACAAGG

        2950
     CAGACATGGAATCTGCACTCGA
```

EP 0 220 520 B1

Fig. 6

# Fig. 7

Fig. 8

# Fig. 9